# EUROPEAN PATENT APPLICATION

(11) **EP 4 255 156 A1**
(43) Date of publication of application: **04.10.2023**
(21) Application number: 22849755.8
(22) Date of filing: 12.07.2022
(51) Int. Cl.: H10K 99/00, H10K 50/00

(54) **ORGANIC LIGHT-EMITTING DEVICE**

(30) Priority: 26.07.2021 KR 20210097800
(71) Applicant: LG Chem, Ltd., Seoul 07336 (KR)
(72) Inventor: SUH, Sang Duk, Daejeon 34122 (KR); JUNG, Min Woo, Daejeon 34122 (KR); LEE, Jungha, Daejeon 34122 (KR); HAN, Su Jin, Daejeon 34122 (KR); PARK, Seulchan, Daejeon 34122 (KR); HWANG, Sunghyun, Daejeon 34122 (KR); LEE, Dong Hoon, Daejeon 34122 (KR)
(74) Representative: Hoffmann Eitle
(86) International application number: PCT/KR2022/010101
(87) International publication number: WO 2023/008779

(57) **Abstract**

The present disclosure provides an organic light emitting device having improved driving voltage, efficiency and lifetime.

## Description

### [TECHNICAL FIELD]

### CROSS-REFERENCE TO RELATED APPLICATION(S)

This application claims the benefit of Korean Patent Application No. 10-2021-0097800 filed on July 26, 2021 with the Korean Intellectual Property Office, the content of which is incorporated herein by reference in its entirety.

The present disclosure relates to an organic light emitting device having improved driving voltage, efficiency and lifetime.

### [BACKGROUND ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuing need for the development of an organic light emitting device having improved driving voltage, efficiency and lifetime, in the organic light emitting device as described above.

### [Prior Art Literature]

### [Patent Literature]

(Patent Literature 1): Korean Unexamined Patent Publication No. 10-2000-0051826

### [DETAILED DESCRIPTION OF THE INVENTION]

### [Technical Problem]

It is an object of the present disclosure to provide an organic light emitting device having improved driving voltage, efficiency and lifetime.

### [Technical Solution]

According to the present disclosure, there is provided an organic light emitting device comprising:
an anode; a cathode; and a light emitting layer interposed between the anode and the cathode,
wherein the light emitting layer includes an organic alloy of a compound represented by the following Chemical Formula 1 and a compound represented by the following Chemical Formula 2. 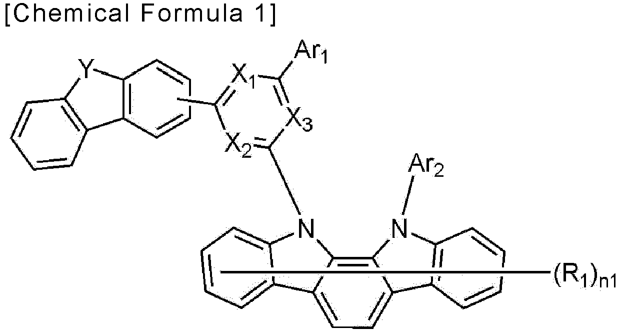 wherein, in Chemical Formula 1,
   X₁, X₂ and X₃ are each independently CH or N, provided that at least one of X₁, X₂ and X₃ is N,
   Ar₁ and Ar₂ are each independently a substituted or unsubstituted C₆₋₆₀ aryl; or a substituted or unsubstituted C₂₋₆₀ heteroaryl containing any one or more selected from the group consisting of N, O and S,
   Y is O, S, or CRR',
   wherein R and R' are each independently a substituted or unsubstituted C₁₋₆₀ alkyl,
   R₁ is hydrogen; deuterium; a substituted or unsubstituted C₆₋₆₀ aryl; or a substituted or unsubstituted C₂₋₆₀ heteroaryl containing at least one selected from the group consisting of N, O and S, and
   n1 is an integer of 1 to 10, 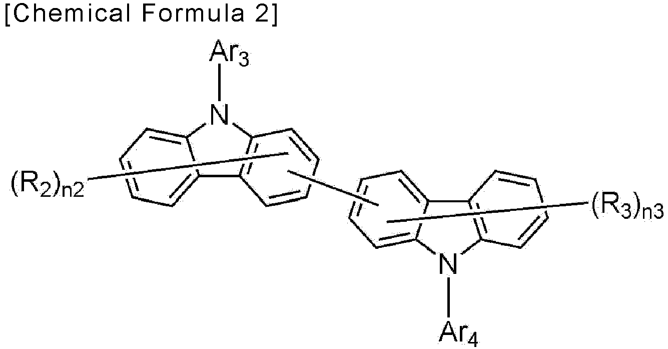 wherein, in Chemical Formula 2,
      Ar₃ and Ar₄ are each independently a substituted or unsubstituted C₆₋₁₂ aryl; or a substituted or unsubstituted C₂₋₁₂ heteroaryl containing any one or more selected from the group consisting of N, O and S,
      R₂ and R₃ are each independently hydrogen; deuterium; a substituted or unsubstituted C₆₋₆₀ aryl; or a substituted or unsubstituted C₂₋₆₀ heteroaryl containing any one or more selected from the group consisting of N, O and S,
      provided that at least one of Ar₃ and Ar₄ is substituted with at least one deuterium, or at least one of R₂ and R₃ is deuterium, and
      n2 and n3 are each independently an integer of 1 to 7.

### [Advantageous Effects]

The above-mentioned organic light emitting device includes an organic alloy of the compound represented by Chemical Formula 1 and the compound represented by Chemical Formula 2 in the light emitting layer, and thus can improve the efficiency, achieve low driving voltage and/or improve lifetime characteristics in the organic light emitting device.

### [BRIEF DESCRIPTION OF THE DRAWINGS]

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, an electron blocking layer 7, a light emitting layer 3, a hole blocking layer 8, an electron transport layer 9, an electron injection layer 10, and a cathode 4.

### [DETAILED DESCRIPTION OF THE EMBODIMENTS]

Hereinafter, embodiments of the present disclosure will be described in more detail to facilitate understanding of the invention.

As used herein, the notation 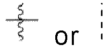 means a bond linked to another substituent group.

As used herein, the term "substituted or unsubstituted" means being unsubstituted or substituted with one or more substituents selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heterocyclic group containing at least one of N, O and S atoms, or being unsubstituted or substituted with a substituent to which two or more substituents of the above-exemplified substituents are connected. For example, "a substituent in which two or more substituents are connected" may be a biphenyl group. Namely, a biphenyl group may be an aryl group, or it may be interpreted as a substituent in which two phenyl groups are connected.

In the present disclosure, the carbon number of a carbonyl group is not particularly limited, but is preferably 1 to 40. Specifically, the carbonyl group may be a substituent having the following structural formulas, but is not limited thereto.

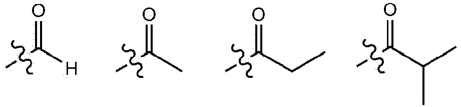

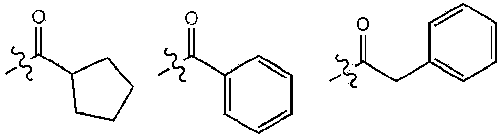

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a substituent having the following structural formulas, but is not limited thereto.

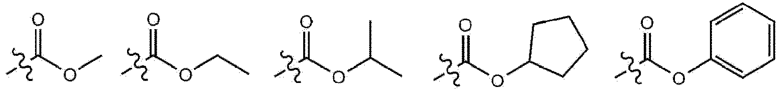

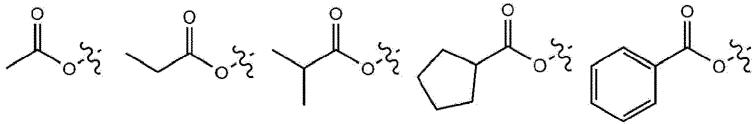

In the present disclosure, the carbon number of an imide group is not particularly limited, but is preferably 1 to 25. Specifically, the imide group may be a substituent having the following structural formulas, but is not limited thereto.

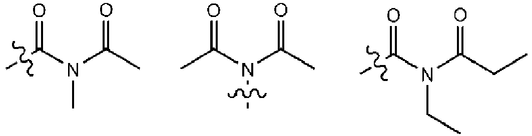

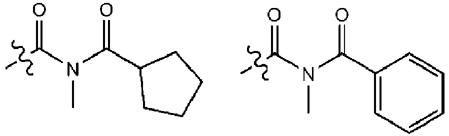

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

In the present disclosure, a boron group specifically includes a trimethylboron group, a triethylboron group, a t-butyldimethylboron group, a triphenylboron group, and a phenylboron group, but is not limited thereto.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methylbutyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The aryl group may be a phenyl group, a biphenyl group, a terphenyl group or the like as the monocyclic aryl group, but is not limited thereto. The polycyclic aryl group includes a naphthyl group, an anthracenyl group, a phenanthrenyl group, a pyrenyl group, a perylenyl group, a chrysenyl group, or the like, but is not limited thereto.

In the present disclosure, the fluorenyl group may be substituted, and two substituents may be linked with each other to form a spiro structure. In the case where the fluorenyl group is substituted, 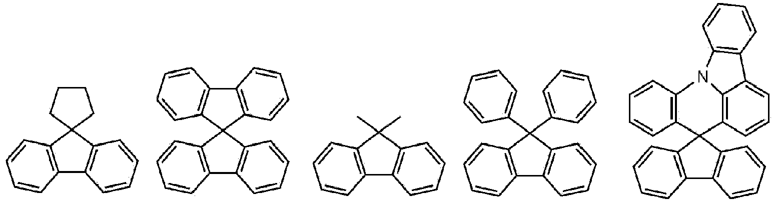 and the like can be formed. However, the structure is not limited thereto.

In the present disclosure, a heterocyclic group is a heterocyclic group containing one or more of O, N, Si and S as a heteroatom, and the carbon number thereof is not particularly limited, but is preferably 2 to 60. Examples of the heterocyclic group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group and the arylamine group is the same as the aforementioned examples of the aryl group. In the present disclosure, the alkyl group in the aralkyl group, the alkylaryl group and the alkylamine group is the same as the aforementioned examples of the alkyl group. In the present disclosure, the heteroaryl in the heteroarylamine can be applied to the aforementioned description of the heterocyclic group. In the present disclosure, the alkenyl group in the aralkenyl group is the same as the aforementioned examples of the alkenyl group. In the present disclosure, the aforementioned description of the aryl group may be applied except that the arylene is a divalent group. In the present disclosure, the aforementioned description of the heterocyclic group can be applied except that the heteroarylene is a divalent group. In the present disclosure, the aforementioned description of the aryl group or cycloalkyl group can be applied except that the hydrocarbon ring is not a monovalent group but formed by combining two substituent groups. In the present disclosure, the aforementioned description of the heterocyclic group can be applied, except that the heterocycle is not a monovalent group but formed by combining two substituent groups.

Meanwhile, in the present disclosure, the organic alloy is a material obtained by pre-treating two or more single organic compounds, and a chemical interaction among the single organic compounds can be provided due to the pre-treatment. The pre-treating may be a heat treatment such as heating and sublimation followed by cooling, but is not limited thereto.

Below, the present disclosure will be described in detail for each configuration.

### Anode and Cathode

An anode and a cathode used in the present disclosure mean electrodes used in an organic light emitting device.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:AL or SnO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

### Hole Injection Layer

The organic light emitting device according to the present disclosure may further include a hole injection layer on the anode, if necessary.

The hole injection layer is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to an electron injection layer or the electron injection material, and further is excellent in the ability to form a thin film. Further, it is preferable that a HOMO (highest occupied molecular orbital) of the hole injection material is between the work function of the anode material and a HOMO of a peripheral organic material layer.

Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive compound, and the like, but are not limited thereto.

### Hole Transport Layer

The organic light emitting device according to the present disclosure may include a hole transport layer on the anode (or on the hole injection layer if the hole injection layer exists), if necessary.

The hole transport layer is a layer that can receive the holes from the anode or the hole injection layer and transport the holes to the light emitting layer, and the hole transport material is suitably a material having large mobility to the holes, which may receive holes from the anode or the hole injection layer and transfer the holes to the light emitting layer.

Specific examples thereof include an arylamine-based organic material, a conductive polymer, a block copolymer in which a conjugate portion and a non-conjugate portion are present together, and the like, but are not limited thereto.

### Electron Blocking Layer

The organic light emitting device according to the present disclosure may include an electron blocking layer on the hole transport layer, if necessary.

The electron blocking layer is a layer provided between the hole transport layer and the light emitting layer in order to prevent the electrons injected in the cathode from being transferred to the hole transport layer without being recombined in the light emitting layer, which may also be referred to as an electron inhibition layer. The electron blocking layer is preferably a material having a smaller electron affinity than the electron transport layer.

### Light Emitting Layer

The light emitting layer used in the present disclosure is a layer that can emit light in the visible light region by combining holes and electrons transported from the anode and the cathode. Generally, the light emitting layer includes a host material and a dopant material, and in the present disclosure, an organic alloy obtained by pretreating the compound represented by Chemical Formula 1 and the compound represented by Chemical Formula 2 may be used as a host.

Preferably, Ar₁ may be a substituted or unsubstituted C₆₋₂₀ aryl, and more preferably, Ar₁ may be phenyl, biphenylyl, terphenylyl, or phenyl substituted with 5 deuteriums. Most preferably, Ar₁ may be any one selected from the group consisting of:

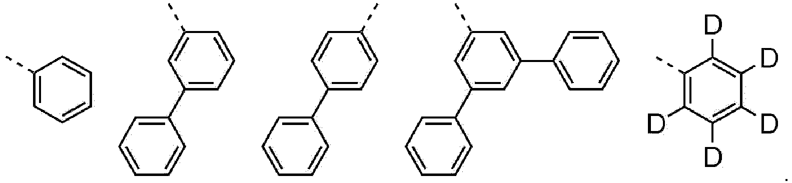

Preferably, Ar₂ may be a substituted or unsubstituted C₆₋₂₀ aryl, and more preferably, Ar₂ may be phenyl, or biphenylyl. Most preferably, Ar₂ may be phenyl or

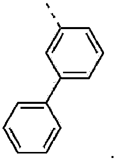

Preferably, R and R' may each independently be a substituted or unsubstituted C₁₋₁₀ alkyl, and more preferably, R and R' may be each independently methyl.

Preferably, R₁ is hydrogen; deuterium; a substituted or unsubstituted C₆₋₂₀ aryl; or a substituted or unsubstituted C₂₋₂₀ heteroaryl containing any one or more selected from the group consisting of N, O and S. More preferably, R₁ may be hydrogen or deuterium.

Representative examples of the compound represented by Chemical Formula 1 are as follows:

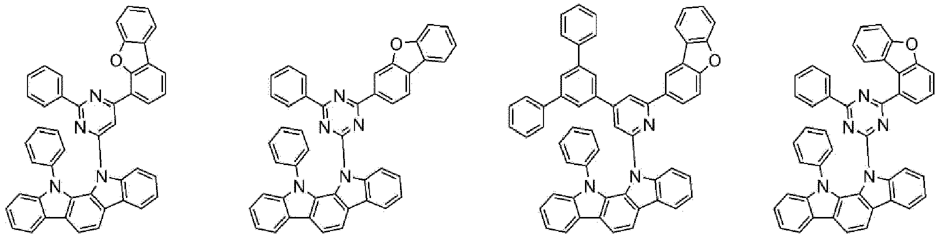

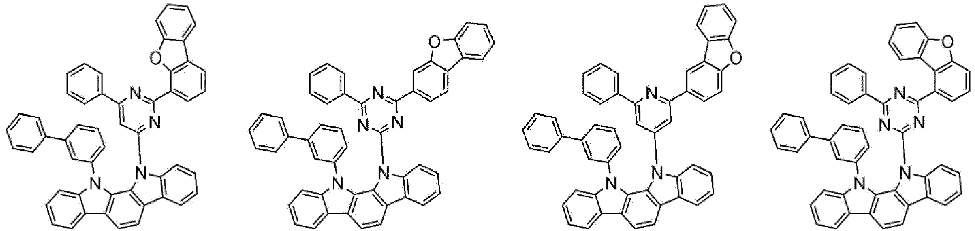

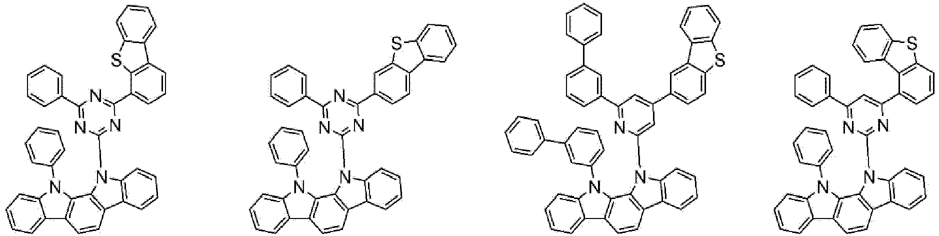

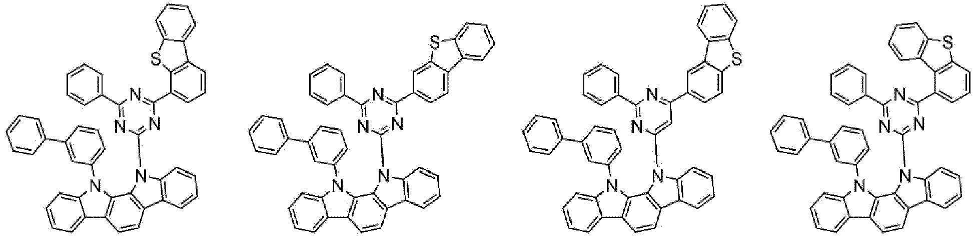

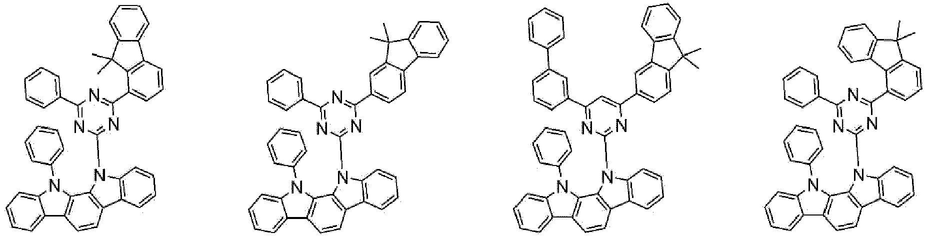

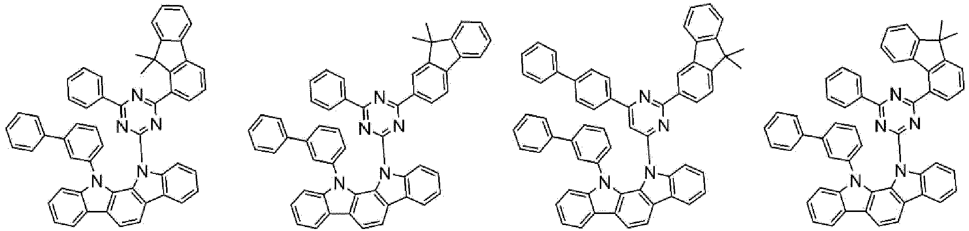

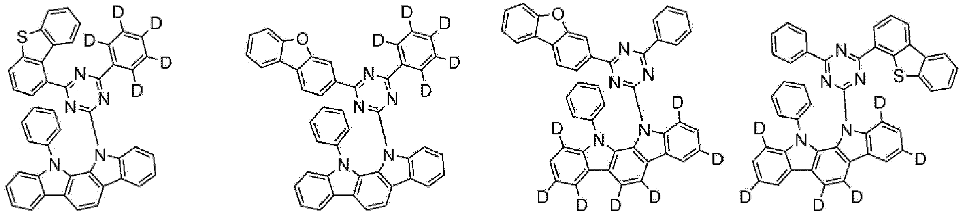

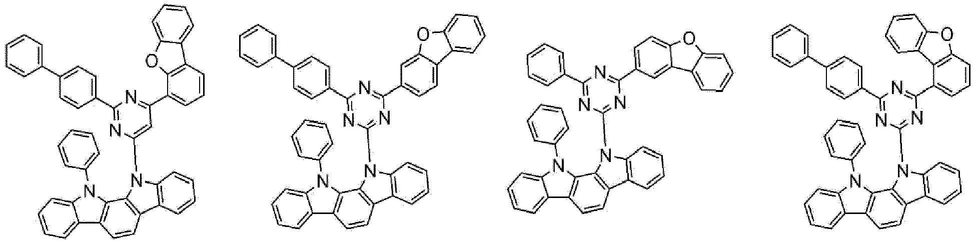

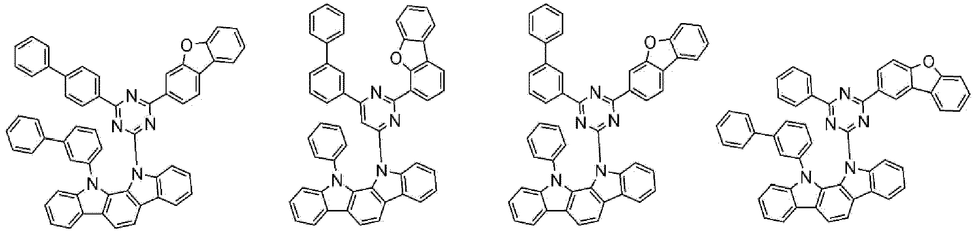

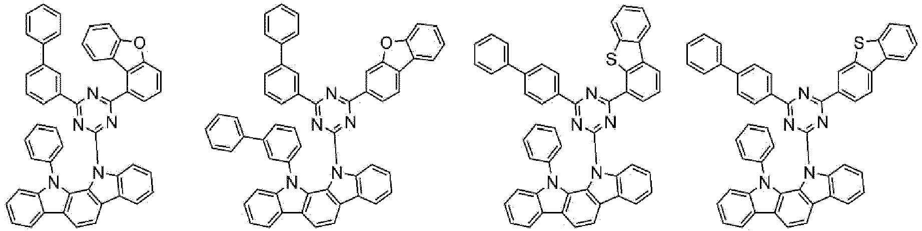

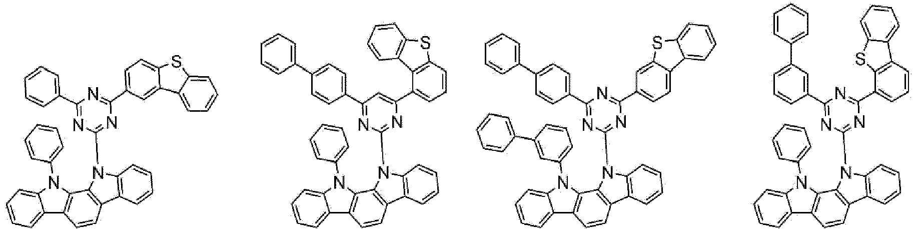

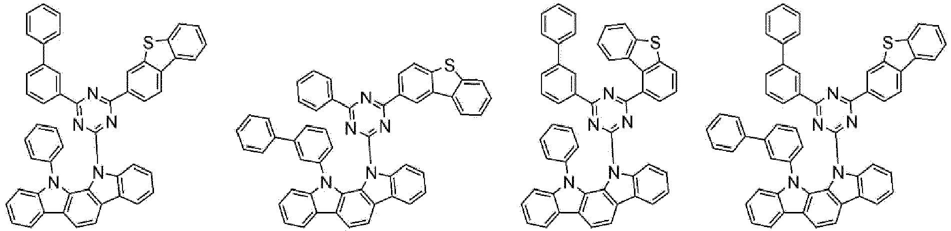

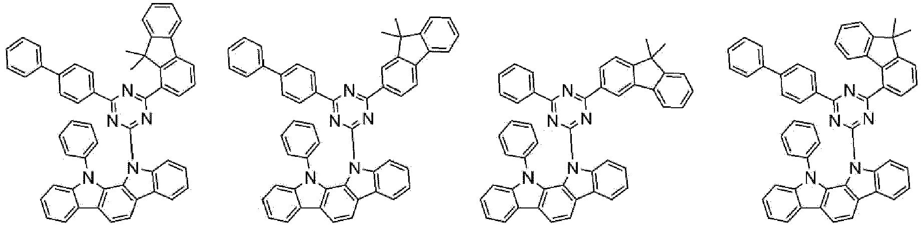

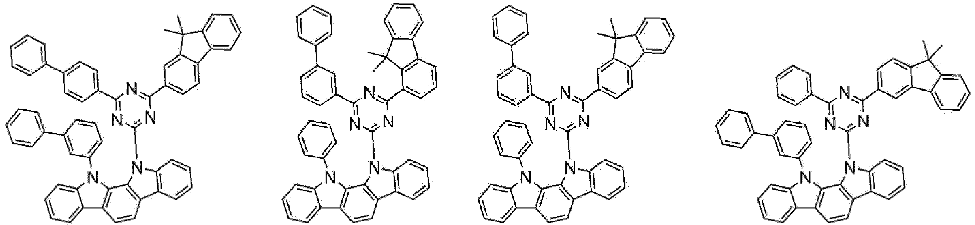

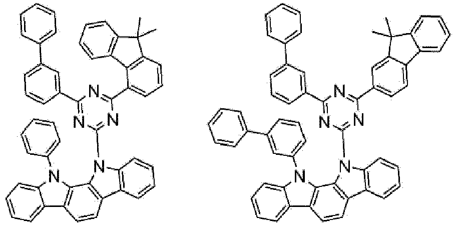

The compound represented by Chemical Formula 1 can be prepared by a preparation method as shown in the following Reaction Scheme 1 as an example, and other remaining compounds can be prepared in a similar manner. 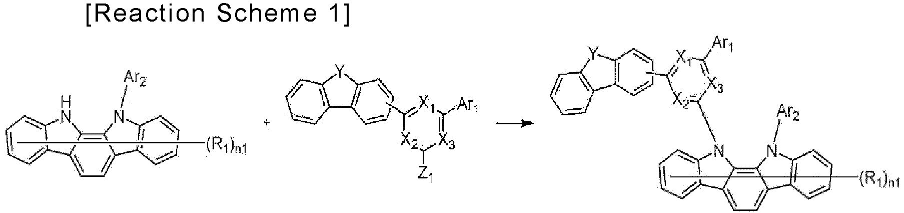 in Reaction Scheme 1, X₁, X₂, X₃, Ar₁, Ar₂, R₁ and n1 are as defined in Chemical Formula 1, and Z₁ is halogen, preferably Z₁ is chloro or bromo.

Reaction Scheme 1 is an amine substitution reaction, which is preferably carried out in the presence of a palladium catalyst and a base, and a reactive group for the amine substitution reaction can be modified as known in the art. The preparation method can be further embodied in Preparation Examples described hereinafter.

Preferably, the compound represented by Chemical Formula 2 may be represented by the following Chemical Formula 2-1: 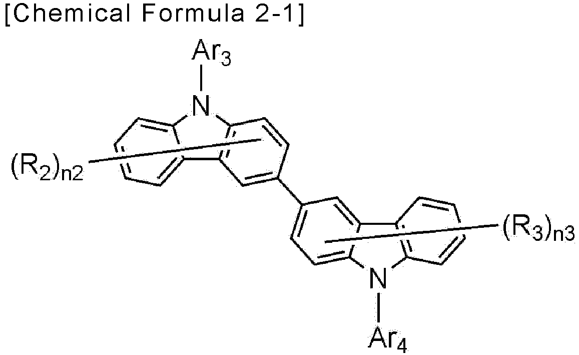 in Chemical Formula 2-1,
Ar₃, Ar₄, R₂, R₃, n2 and n3 are as defined in Chemical Formula 2.

Preferably, Ar₃ and Ar₄ may be each independently phenyl, biphenylyl, naphthyl, dimethylfluorenyl, dibenzofuranyl, or dibenzothiophenyl, wherein the phenyl, biphenylyl, naphthyl, dimethylfluorenyl, dibenzofuranyl, or dibenzothiophenyl may be unsubstituted or substituted with deuterium (D).

R₂ and R₃ may be each independently hydrogen; deuterium; a substituted or unsubstituted C₆₋₂₀ aryl; or a substituted or unsubstituted C₂₋₂₀ heteroaryl containing any one or more selected from the group consisting of N, O and S. More preferably, R₂ and R₃ may be each independently hydrogen, deuterium, or phenyl, wherein the phenyl may be unsubstituted or substituted with 1 to 5 deuteriums.

Representative examples of the compound represented by Chemical Formula 2 are as follows: 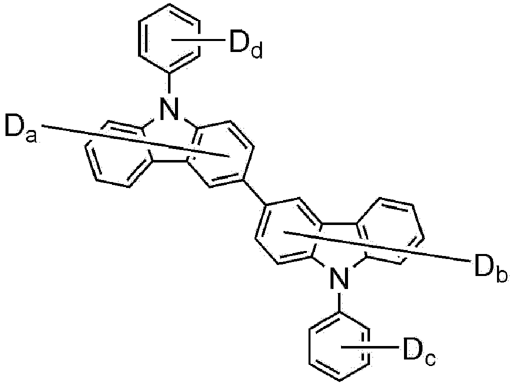 in the above compound,
a + b + c + d is 1 to 24, 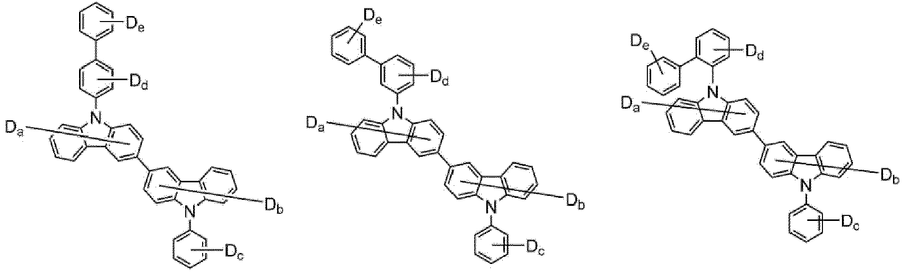 in the above group,
a + b + c + d + e is 1 to 28, 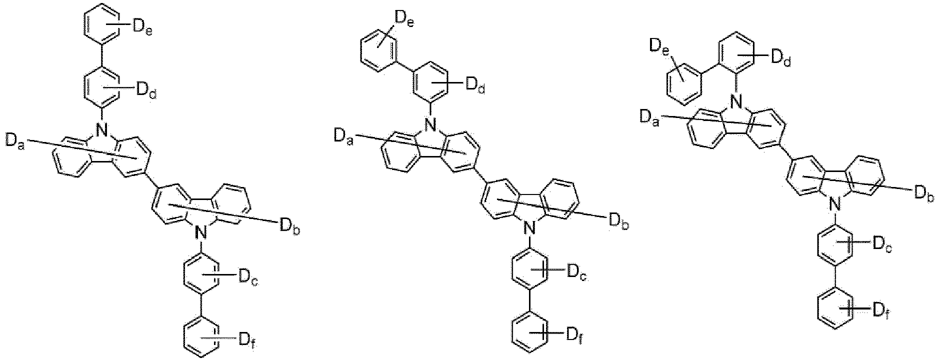 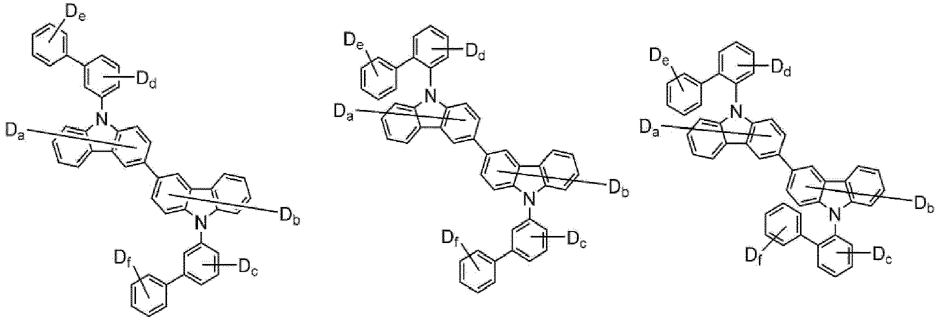 in the above group,
a + b + c + d + e + fis1 to 32, 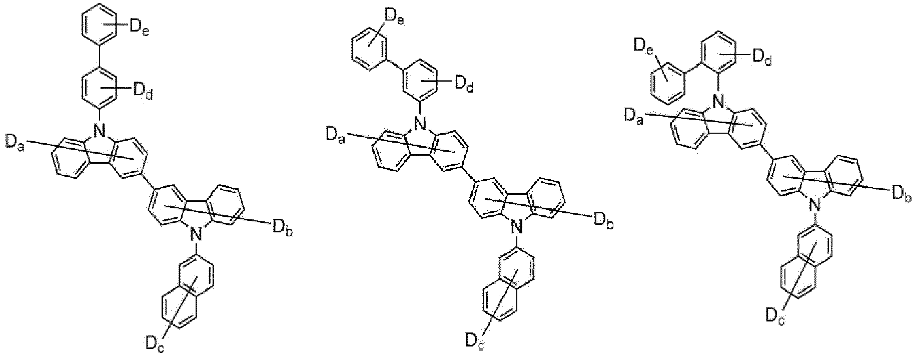 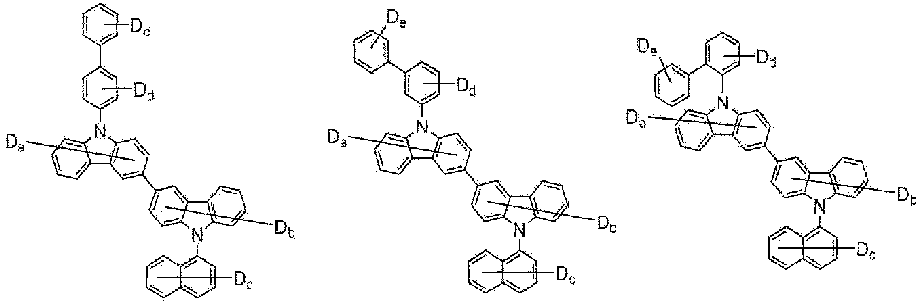 in the above group,
a + b + c + d + e is 1 to 30, 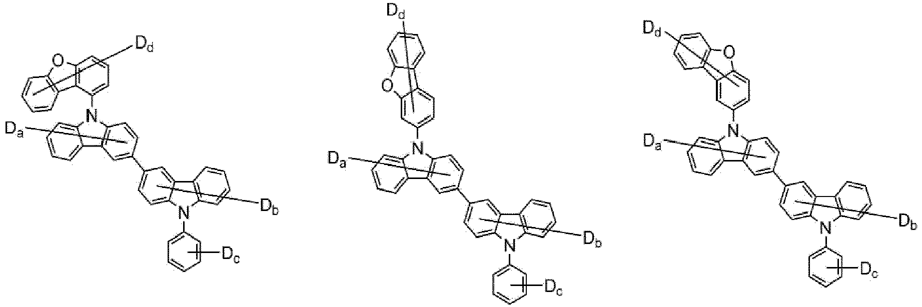 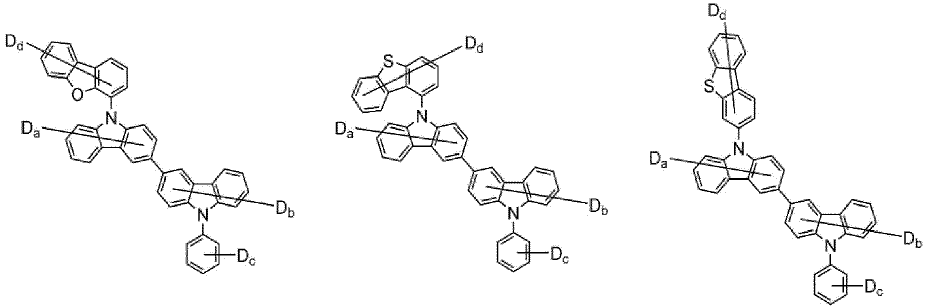 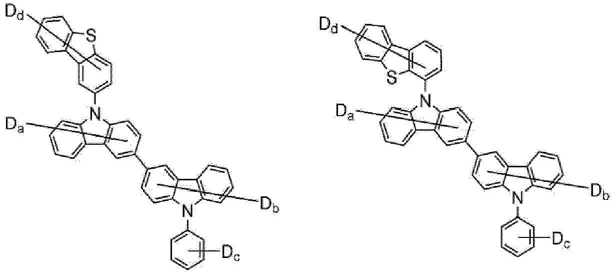 in the above group,
a + b + c + d is 1 to 26, 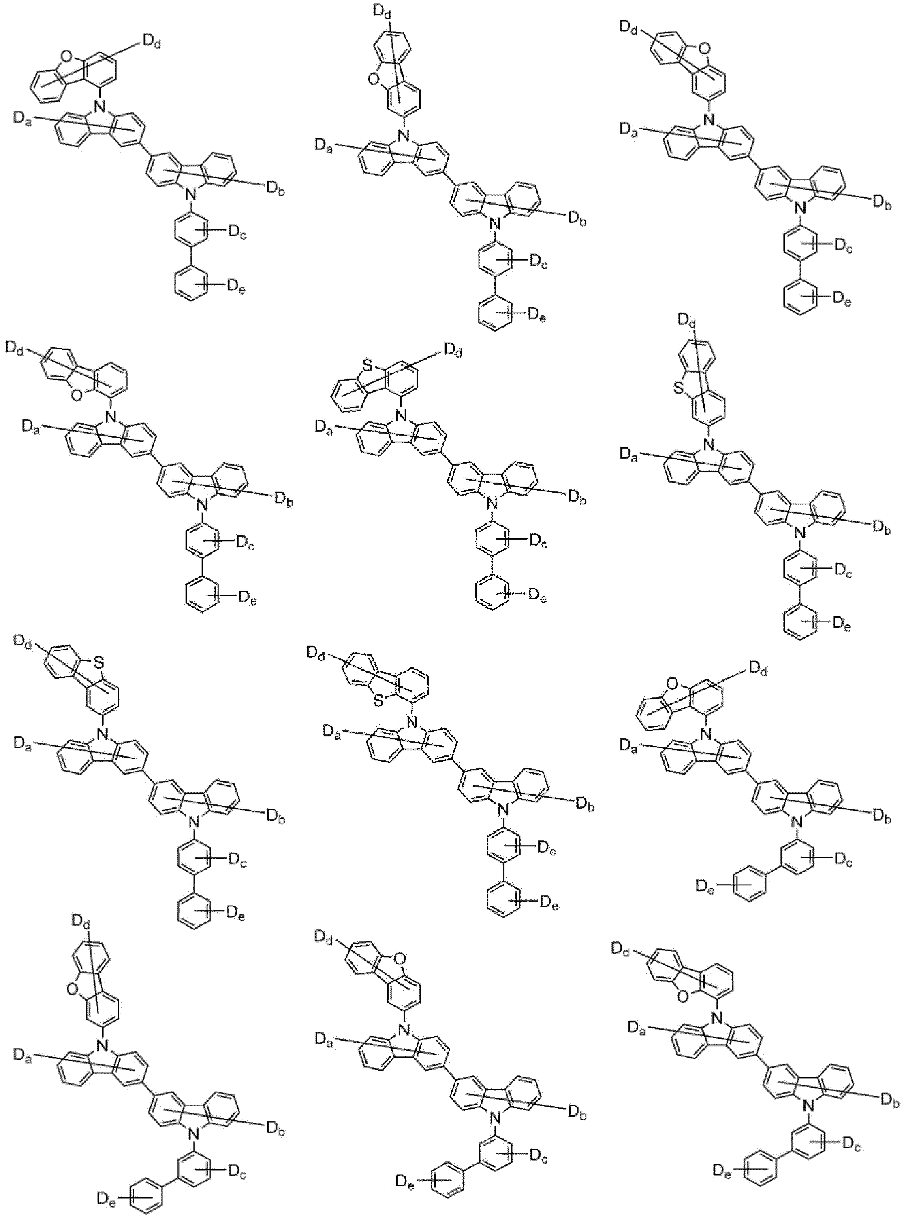 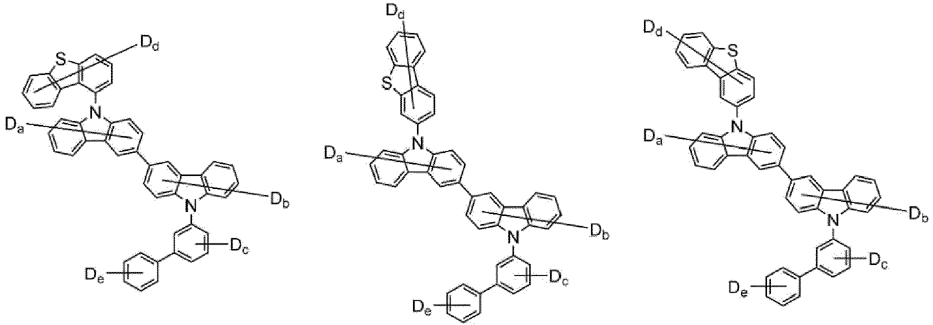 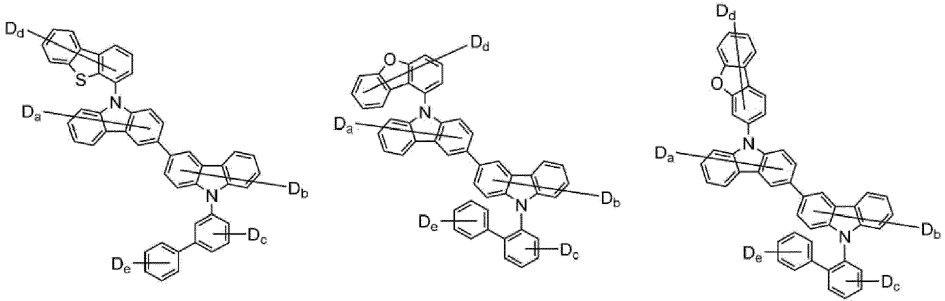 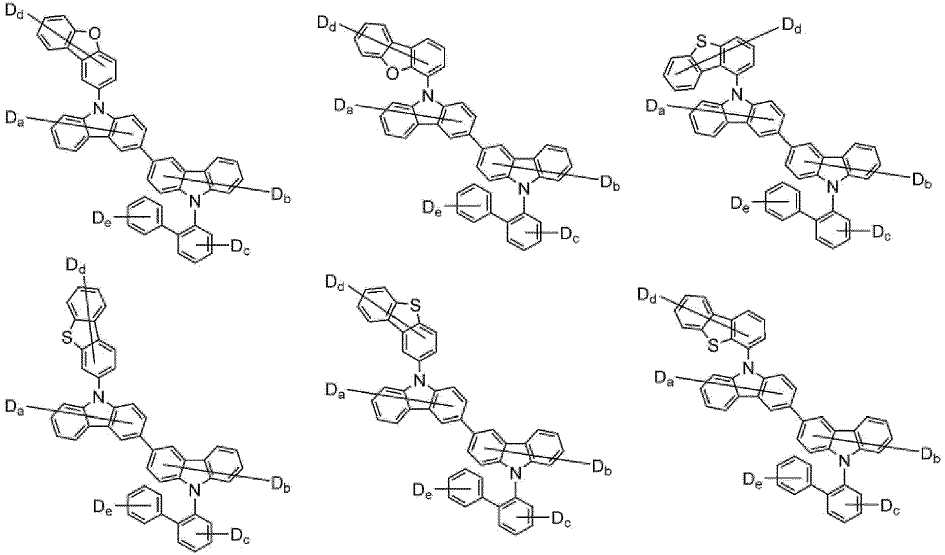 in the above group,
a + b + c + d + e is 1 to 30, 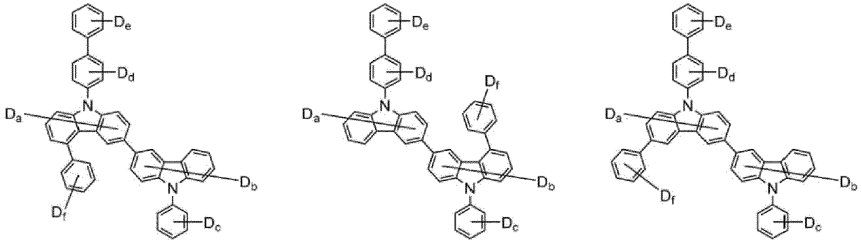 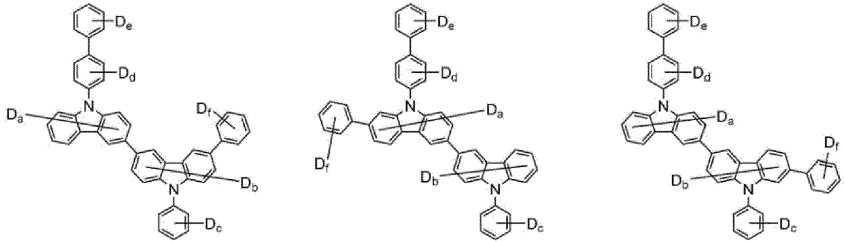 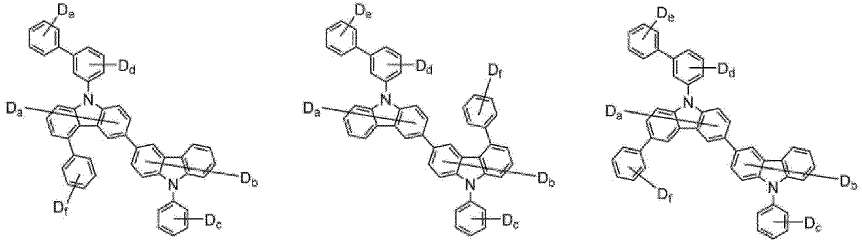 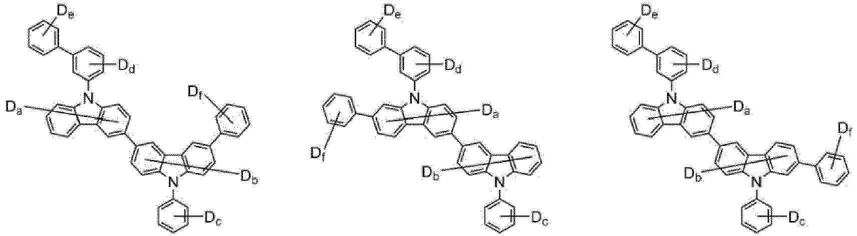 in the above group,
a + b + c + d + e + fis1 to 32, 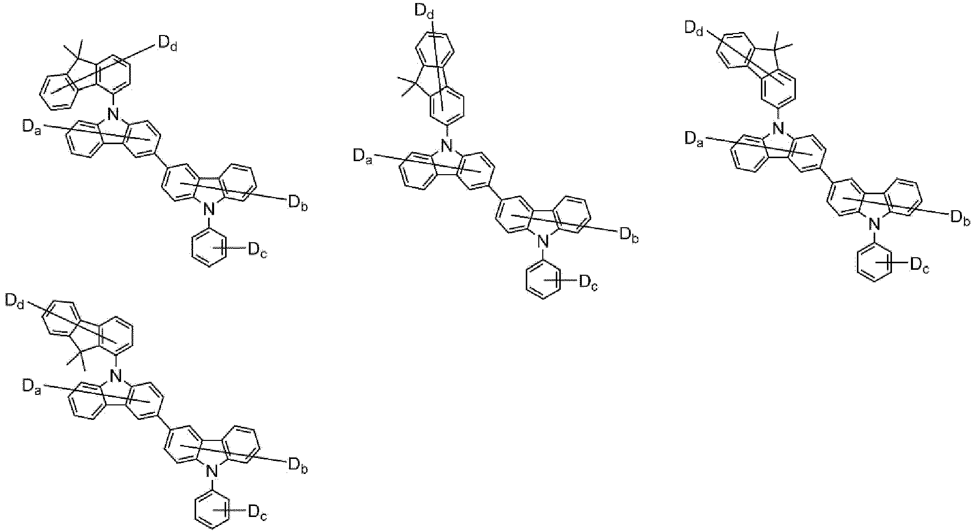 in the above group,
a + b + c + d is 1 to 32, 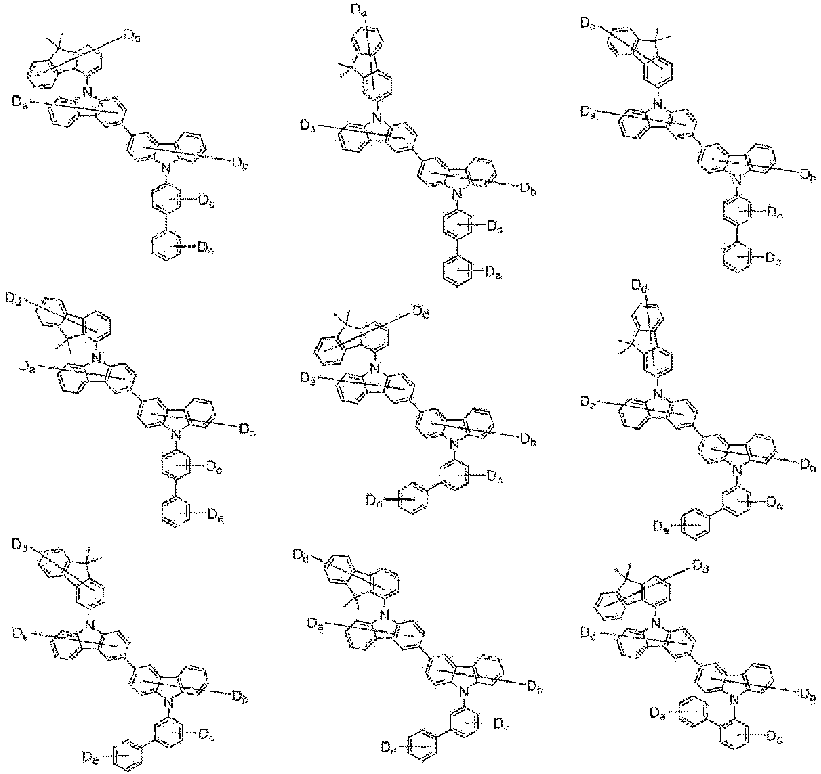 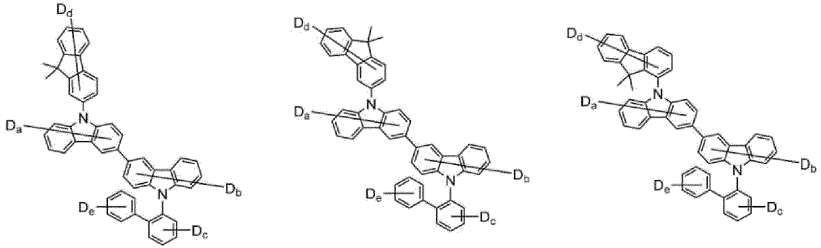 in the above group,
a + b + c + d + e is 1 to 36.

The compound represented by Chemical Formula 2 can be prepared by a preparation method as shown in the following Reaction Scheme 2 as an example, and the other remaining compounds can be prepared in a similar manner. 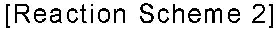 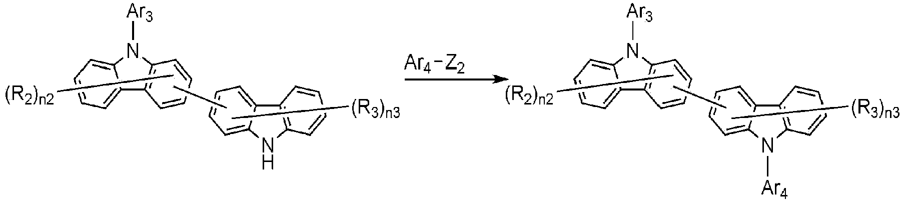 in Reaction Scheme 2, Ar₃, Ar₄, R₂, R₃, n2 and n3 are as defined in Chemical Formula 2, and Z₂ is halogen, preferably Z₂ is chloro or bromo.

Reaction Scheme 2 is an amine substitution reaction, which is preferably carried out in the presence of a palladium catalyst and a base, and a reactive group for the amine substitution reaction can be modified as known in the art. The preparation method can be further embodied in Preparation Examples described hereinafter.

Preferably, in the organic alloy, the weight ratio between the compound represented by Chemical Formula 1 to the compound represented by Chemical Formula 2 is 10:90 to 90:10, more preferably 20:80 to 80:20, 30:70 to 70:30 or 40:60 to 60:40.

Meanwhile, the light emitting layer may further include a dopant in addition to the host. The dopant material is not particularly limited as long as it is a material used for the organic light emitting device. As an example, an aromatic amine derivative, a styrylamine compound, a boron complex, a fluoranthene compound, a metal complex, and the like can be mentioned. Specific examples of the aromatic amine derivatives include substituted or unsubstituted fused aromatic ring derivatives having an arylamino group, examples thereof include pyrene, anthracene, chrysene, and periflanthene having the arylamino group, and the like. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, examples of the metal complex include an iridium complex, a platinum complex, and the like, but are not limited thereto.

### Hole Blocking Layer

The organic light emitting device according to the present disclosure may include a hole blocking layer on the light emitting layer, if necessary.

The hole blocking layer is a layer provided between the electron transport layer and the light emitting layer in order to prevent the holes injected in the anode from being transferred to the electron transport layer without being recombined in the light emitting layer, which may also be referred to as a hole stopping layer. The hole blocking layer is preferably a material having high ionization energy.

### Electron Transport Layer

The organic light emitting device according to the present disclosure may include an electron transport layer on the light emitting layer, if necessary.

The electron transport layer is a layer that receives the electrons from the electron injection layer formed on the cathode or the cathode and transports the electrons to the light emitting layer, and that suppress the transfer of holes from the light emitting layer, and an electron transport material is suitably a material which may receive electrons well from a cathode and transfer the electrons to a light emitting layer, and has a large mobility for electrons.

Specific examples of the electron transport material include: an Al complex of 8-hydroxyquinoline; a complex including Alqs; an organic radical compound; a hydroxyflavone-metal complex, and the like, but are not limited thereto. The electron transport layer may be used with any desired cathode material, as used according to a conventional technique. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

### Electron Injection Layer

The organic light emitting device according to the present disclosure may further include an electron injection layer on the light emitting layer (or on the electron transport layer, if the electron transport layer exists).

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film.

Specific examples of the electron injection layer include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

### Organic Light Emitting Device

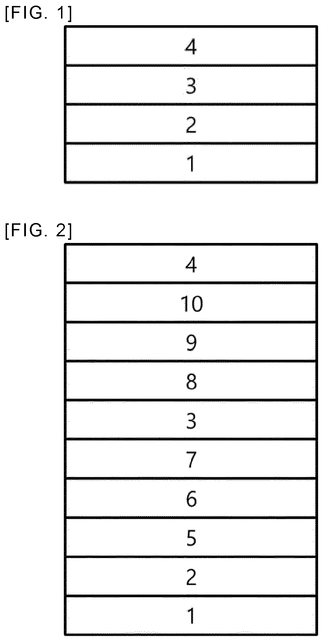

The structure of the organic light emitting device according to the present disclosure is illustrated in FIGS. 1 and 2. FIG. 1 shows an example of an organic light emitting device comprising a substrate 1, an anode 2, a light emitting layer 3, and a cathode 4. FIG. 2 shows an example of an organic light emitting device comprising a substrate 1, an anode 2, a hole injection layer 5, a hole transport layer 6, an electron blocking layer 7, a light emitting layer 3, a hole blocking layer 8, an electron transport layer 9, an electron injection layer 10, and a cathode 4.

The organic light emitting device according to the present disclosure can be manufactured by sequentially stacking the above-described structures. In this case, the organic light emitting device may be manufactured by depositing a metal, metal oxides having conductivity, or an alloy thereof on the substrate by using a PVD (physical vapor deposition) method such as a sputtering method or an e-beam evaporation method to form the anode, forming the respective layers described above thereon, and then depositing a material that can be used as the cathode thereon. In addition to such a method, the organic light emitting device may be manufactured by sequentially depositing from the cathode material to the anode material on a substrate in the reverse order of the above-mentioned configuration (WO 2003/012890). Further, the light emitting layer may be formed by subjecting hosts and dopants to a vacuum deposition method and a solution coating method. Herein, the solution coating method means a spin coating, a dip coating, a doctor blading, an inkjet printing, a screen printing, a spray method, a roll coating, or the like, but is not limited thereto.

Meanwhile, the organic light emitting device according to the present disclosure may be a bottom emission device, a top emission device, or a double-sided light emitting device, and particularly, may be a bottom emission device that requires relatively high luminous efficiency.

Below, the embodiments are described in more detail to assist in the understanding of the present disclosure. However, the following examples are provided for illustrative purposes only, and are not intended to limit the subject matter of the present disclosure.

### [Preparation Example 1: Preparation of Compound]

### Preparation Example 1-1: Synthesis of Compound 1-1

### Step 1) Synthesis of Compound 1-1-a

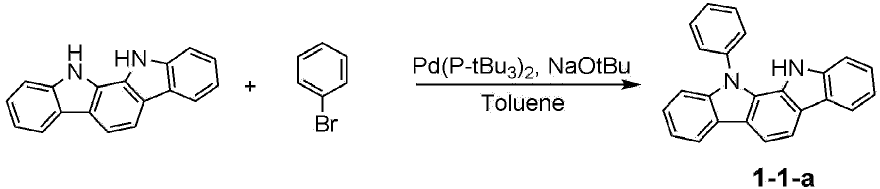

11,12-Dihydroindolo[2,3-a]carbazole (15.0 g, 58.5 mmol) and bromobenzene (10.1 g, 64.4 mmol) were added to 300 ml of toluene under a nitrogen atmosphere, and the mixture was stirred and refluxed. Then, sodium tert-butoxide (8.4 g, 87.8 mmol) and bis(tri-tert-butylphosphine) palladium (0) (0.9 g, 1.8 mmol) were added thereto. After the reaction for 10 hours, the reaction mixture was cooled to room temperature, the organic layer was separated using chloroform and water, and then the organic layer was distilled. This was dissolved again in chloroform, washed twice with water, and the organic layer was separated. Anhydrous magnesium sulfate was added, and the mixture was stirred, then filtered, and the filtrate was distilled under reduced pressure. The concentrated compound was purified by silica gel column chromatography to prepare 12.8 g of Compound 1-1-a. (Yield: 66%, MS: [M+H]⁺= 333).

### Step 2) Synthesis of Compound 1-1

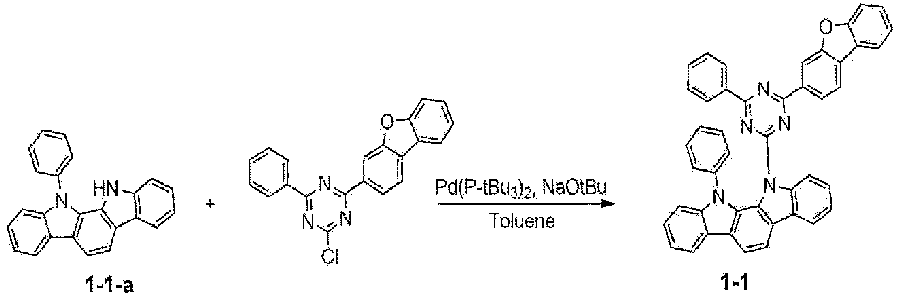

Compound 1-1-a (15.0 g, 45.1 mmol) and 2-chloro-4-(dibenzo[b,d]furan-3-yl)-6-phenyl-1,3,5-triazine (17.8 g, 49.6 mmol) were added to 300 ml of toluene under a nitrogen atmosphere, and the mixture was stirred and refluxed. Then, sodium tert-butoxide (6.5 g, 67.7 mmol) and bis(tri-tert-butylphosphine) palladium (0) (0.7 g, 1.4 mmol ) were added thereto. After the reaction for 12 hours, the reaction mixture was cooled to room temperature, the organic layer was separated using chloroform and water, and then the organic layer was distilled. This was dissolved again in chloroform, washed twice with water, and the organic layer was separated. Anhydrous magnesium sulfate was added, and the mixture was stirred, then filtered, and the filtrate was distilled under reduced pressure. The concentrated compound was purified by silica gel column chromatography to prepare 14.5 g of Compound 1-1. (Yield: 49%, MS: [M+H]⁺= 747).

### Preparation Example 1-2: Synthesis of Compound 1-2

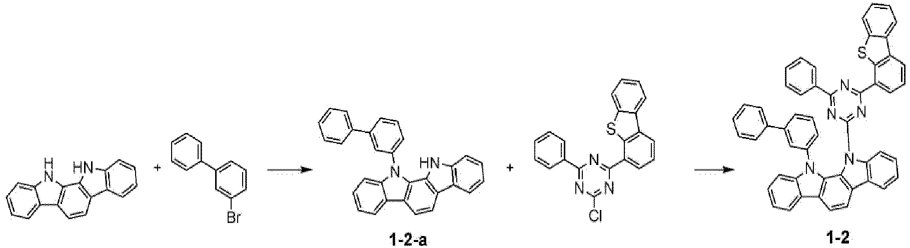

Compound 1-2 (MS[M+H]⁺= 757) was prepared in the same manner as in Preparation Example 1-1, except that in Preparation Example 1-1, bromobenzene was changed to 3-bromo-1 ,1'-biphenyl, and 2-chloro-4-(dibenzo[b,d]furan-3-yl)-6-phenyl-1,3,5-triazine was changed to 2-chloro-4-(dibenzo[b,d]thiophen-4-yl)-6-phenyl-1,3,5-triazine.

### Preparation Example 1-3: Synthesis of Compound 1-3

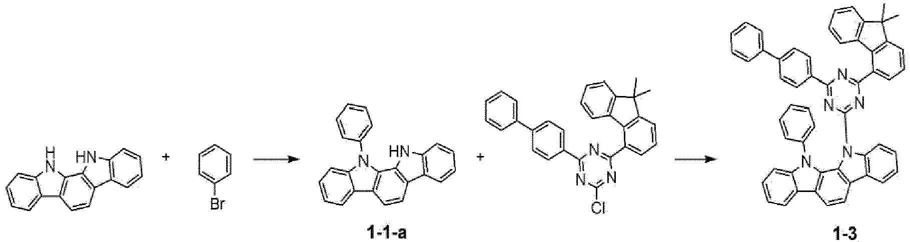

Compound 1-3 (MS[M+H]⁺=717) was prepared in the same manner as in Preparation Example 1-1, except that in Preparation Example 1-1, 2-chloro-4-(dibenzo[b,d]furan-3-yl)-6-phenyl-1,3,5-triazine was changed to 2-([1,1'-biphenyl]-4-yl)-4-chloro-6-(9,9-dimethyl-9H-fluoren-4-yl)-1,3,5-triazine.

### Preparation Example 1-4: Synthesis of Compound 1-4

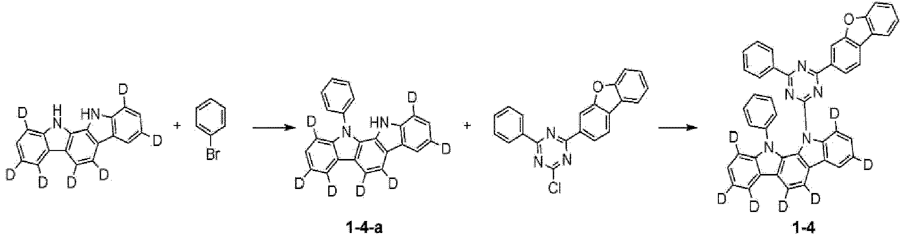

Compound 1-4 (MS[M+H]⁺= 662) was prepared in the same manner as in Preparation Example 1-1, except that in Preparation Example 1-1, 11,12-dihydroindolo[2,3-a]carbazole was changed to 11,12-dihydroindolo[2,3-a]carbazole-1,3,4,5,6,8,10-d7.

### Preparation Example 1-5: Synthesis of Compound 2-1

### Step 1) Synthesis of Compound 2-1-a

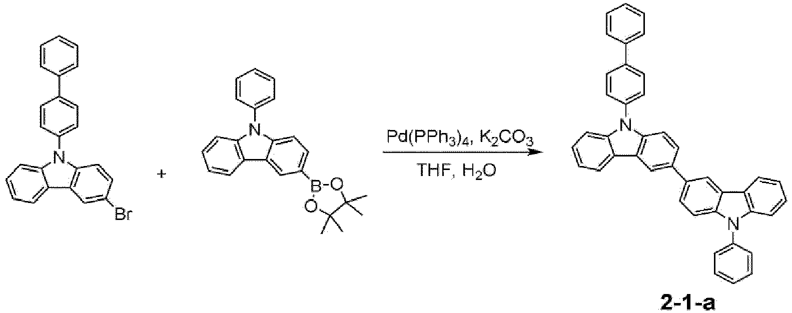

9-([1,1'-biphenyl]-4-yl)-3-bromo-9H-carbazole (15.0 g, 37.7 mmol) and 9-phenyl-3-(4,4,5,5-tetramethyl-1,3,2-dioxaborolan-2-yl)-9H-carbazole (15.3 g, 41.4 mmol) were added to 300 ml of THF under a nitrogen atmosphere, and the mixture was stirred and refluxed. Then, potassium carbonate (20.8 g, 150.6 mmol) was dissolved in 62ml of water and added thereto, and the mixture was sufficiently stirred and then tetrakis(triphenylphosphine)palladium(0)(1.3 g, 1.1 mmol) was added. After the reaction for 9 hours, the reaction mixture was cooled to room temperature, and the organic layer and the aqueous layer were separated and then the organic layer was distilled. This was again dissolved in chloroform, washed twice with water, and then the organic layer was separated, anhydrous magnesium sulfate was added thereto, stirred, and then filtered, and the filtrate was distilled under reduced pressure. The concentrated compound was purified by silica gel column chromatography to prepare 13.5g of Compound 2-1-a. (Yield: 64%, MS: [M+H]+= 562)

### Step 2) Synthesis of Compound 2-1

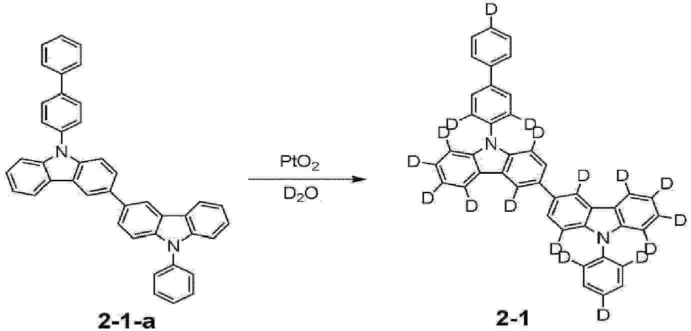

Compound 2-1-a (10.0 g, 17.8 mmol), PtO₂ (1.2 g, 5.4 mmol) and D₂O (89 ml) were added to a shaker tube, and then the tube was sealed and heated at 250°C and 600 psi for 12 hours. When the reaction was completed, chloroform was added, and the reaction solution was transferred to a separatory funnel, and extracted. The extract was dried over anhydrous magnesium sulfate and concentrated, and then the sample was purified by silica gel column chromatography and then subjected to sublimation purification to prepare 3.9 g of Compound 2-1. (Yield: 38%, MS[M+H]⁺= 580)

### Preparation Example 1-6: Synthesis of Compound 2-2

### Step 1) Synthesis of Compound 2-2-a

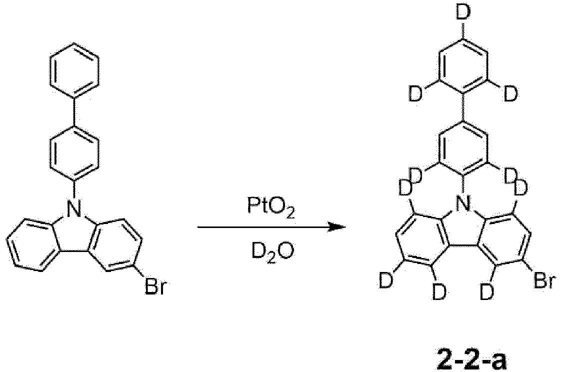

9-([1,1'-Biphenyl]-4-yl)-3-bromo-9H-carbazole (10 g, 25.1 mmol), PtO₂(1.7 g, 7.5 mmol) and D₂O (126 ml) were added to a shaker tube, and then the tube was sealed and heated at 250°C and 600 psi for 12 hours. When the reaction was completed, chloroform was added, and the reaction solution was transferred to a separatory funnel, and extracted. The extract was dried over anhydrous magnesium sulfate and concentrated, and then the sample was purified by silica gel column chromatography to prepare 7.9 g of Compound 2-2-a. (Yield: 77%, MS: [M+H]⁺= 409)

### Step 2) Synthesis of Compound 2-2

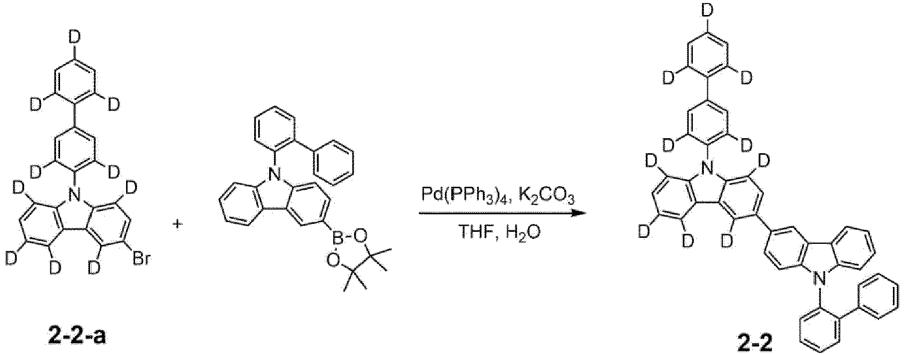

Compound 2-2-a (15.0 g, 36.7 mmol) and 9-([1,1'-biphenyl]-2-yl)-3-(4,4,5,5-tetramethyl-1,3,2-dioxaborolan-2-yl)-9H-carbazole (18.0 g, 40.4 mmol) were added to 300 ml of THF under a nitrogen atmosphere, and the mixture was stirred and refluxed. Then, potassium carbonate (20.3 g, 146.9 mmol) was dissolved in 61ml of water and added thereto, and the mixture was sufficiently stirred and then tetrakis(triphenylphosphine)palladium (0) (1.3 g, 1.1 mmol) was added. After the reaction for 12 hours, the reaction mixture was cooled to room temperature, and the organic layer and the aqueous layer were separated and then the organic layer was distilled. This was again dissolved in chloroform, washed twice with water, and then the organic layer was separated, anhydrous magnesium sulfate was added thereto, stirred, and then filtered, and the filtrate was distilled under reduced pressure. The concentrated compound was purified by silica gel column chromatography and then subjected to sublimation purification to prepare 11.6 g of Compound 2-2. (Yield: 49%, MS: [M+H]⁺= 648)

### Preparation Example 1-7: Synthesis of Compound 2-3

### Step 1) Synthesis of Compound 2-3-a

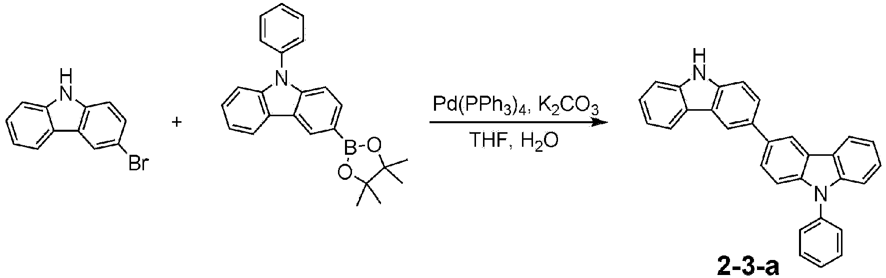

3-Bromo-9H-carbazole (15.0 g, 60.9 mmol) and 9-phenyl-3-(4,4,5,5-tetramethyl-1,3,2-dioxaborolan-2-yl)-9H-carbazole (24.8 g, 67 mmol) were added to 300 ml of THF under a nitrogen atmosphere, and the mixture was stirred and refluxed. Then, potassium carbonate (33.7 g, 243.8 mmol) was dissolved in 101ml of water and added thereto, and the mixture was sufficiently stirred and then tetrakis(triphenylphosphine)palladium (0) (2.1 g, 1.8 mmol) was added. After the reaction for 11 hours, the reaction mixture was cooled to room temperature, and the organic layer and the aqueous layer were separated and then the organic layer was distilled. This was again dissolved in chloroform, washed twice with water, and then the organic layer was separated, anhydrous magnesium sulfate was added thereto, stirred, and then filtered, and the filtrate was distilled under reduced pressure. The concentrated compound was purified by silica gel column chromatography to prepare 16.7g of Compound 2-3-a. (Yield: 67%, MS: [M+H]⁺= 410)

### Step 2) Synthesis of Compound 2-3-b

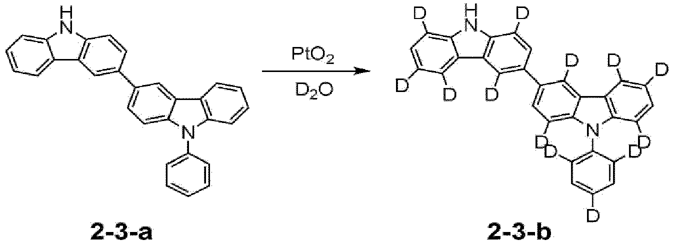

Compound 2-3-a (10.0 g, 24.5 mmol), PtO₂(1.7 g, 7.3 mmol) and D₂O (122 ml) were added to a shaker tube, and then the tube was sealed and heated at 250°C and 600 psi for 12 hours. After completion of the reaction, chloroform was added, and the reaction solution was transferred to a separatory funnel, and extracted. The extract was dried over anhydrous magnesium sulfate and concentrated, and then the sample was purified by silica gel column chromatography to prepare 9.1 g of Compound 2-3-b. (Yield: 88%, MS: [M+H]⁺= 423)

### Step 3) Synthesis of Compound 2-3

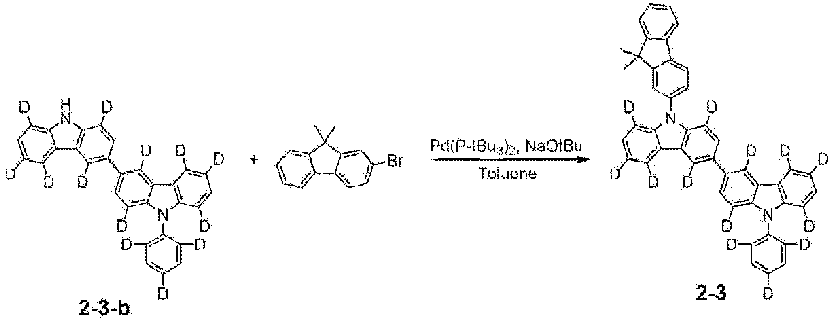

Compound 2-3-b (15.0 g, 36.7 mmol) and 2-bromo-9,9-dimethyl-9H-fluorene(11.0 g, 40.4 mmol) were added to 300 ml of toluene under a nitrogen atmosphere, and the mixture was stirred and refluxed. Then, sodium tert-butoxide (5.3 g, 55.1 mmol) and bis(tri-tert-butylphosphine) palladium (0) (0.6 g, 1.1 mmol) were added thereto. After the reaction for 6 hours, the reaction mixture was cooled to room temperature, the organic layer was separated using chloroform and water, and then the organic layer was distilled. This was dissolved again in chloroform, washed twice with water, and the organic layer was separated. Anhydrous magnesium sulfate was added, and the mixture was stirred, then filtered, and the filtrate was distilled under reduced pressure. The concentrated compound was purified by silica gel column chromatography and then subjected to sublimation purification to prepare 9.5 g of Compound 2-3. (Yield: 42%, MS: [M+H]⁺= 615).

### Preparation Example 1-8: Synthesis of Compound 2-4

### Step 1) Synthesis of Compound 2-4-a

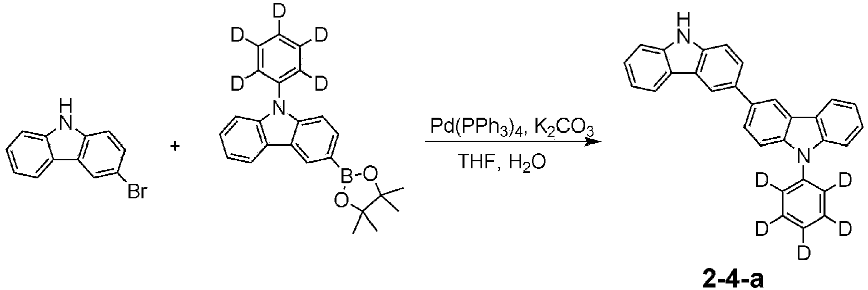

3-Bromo-9H-carbazole (15.0 g, 60.9 mmol) and 9-(phenyl-d5)-3-(4,4,5,5-tetramethyl-1 ,3,2-dioxaborolan-2-yl)-9H-carbazole (25.1 g, 67 mmol) were added to 300 ml of THF under a nitrogen atmosphere, and the mixture was stirred and refluxed. Then, potassium carbonate (33.7 g, 243.8 mmol) was dissolved in 101ml of water and added thereto, and the mixture was sufficiently stirred and then tetrakis(triphenylphosphine) palladium (0) (2.1 g, 1.8 mmol) was added. After the reaction for 9 hours, the reaction mixture was cooled to room temperature, and the organic layer and the aqueous layer were separated and then the organic layer was distilled. This was again dissolved in chloroform, washed twice with water, and then the organic layer was separated, anhydrous magnesium sulfate was added thereto, stirred, and then filtered, and the filtrate was distilled under reduced pressure. The concentrated compound was purified by silica gel column chromatography to prepare 16.1g of Compound 2-4-a. (Yield: 64%, MS: [M+H]⁺= 415)

### Step 2) Synthesis of Compound 2-4

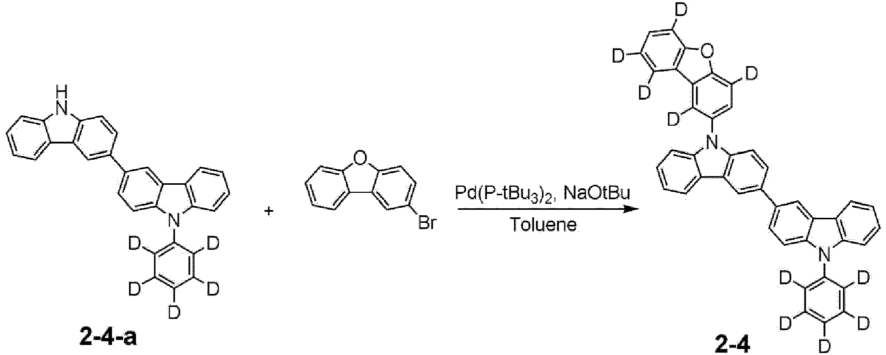

Compound 2-4-a (15.0 g, 36.3 mmol) and 2-bromodibenzo[b,d]furan(9.9 g, 39.9 mmol) were added to 300 ml of toluene under a nitrogen atmosphere, and the mixture was stirred and refluxed. Then, sodium tert-butoxide (5.2 g, 54.4 mmol) and bis(tri-tert-butylphosphine) palladium (0) (0.6 g, 1.1 mmol) were added thereto. After the reaction for 11 hours, the reaction mixture was cooled to room temperature, the organic layer was separated using chloroform and water, and then the organic layer was distilled. This was dissolved again in chloroform, washed twice with water, and the organic layer was separated. Anhydrous magnesium sulfate was added, and the mixture was stirred, then filtered, and the filtrate was distilled under reduced pressure. The concentrated compound was purified by silica gel column chromatography to prepare 8.1 g of Compound 2-4. (Yield: 38%, MS: [M+H]⁺= 586).

### [Preparation Example 2: Preparation of Organic Alloy]

### Preparation Example 2-1: Preparation of Organic Alloy 1

Compound 1-1 and Compound 2-1 were mixed and placed in a weight ratio of 40:60 in a vacuum chamber. The two mixtures were melted by increasing the temperature under a pressure of 10⁻² Torr or less, and then the mixture was cooled to room temperature after 1 hour to obtain a solid product. This product was ground with a mixer to obtain a powdery organic alloy 1.

### Preparation Examples 2-2 to 2-5 and Preparation Examples 2-A to 2-D

Organic alloys 2 to 5 and organic alloys A to D were prepared in the same manner as in Preparation Example 2-1, except that the materials to be mixed were changed as shown in Table 1 below. Compounds A-1, A-2, B-1 and B-2 of Table 1 below are as follows.

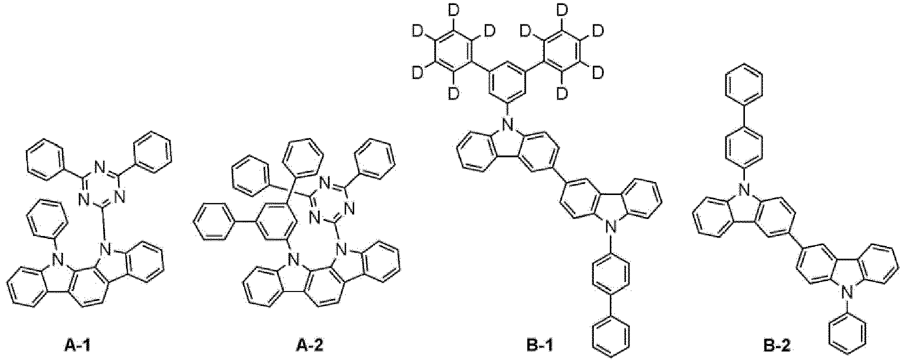

**[Table 1]**

| Preparation Example | Organic alloy | Mixed material 1 | Mixed material 2 | Mixing ratio (weight ratio) |
|---|---|---|---|---|
| Preparation Example 2-1 | Organic alloy 1 | Compound 1-1 | Compound 2-1 | 40:60 |
| Preparation Example 2-2 | Organic alloy 2 | Compound 1-2 | Compound 2-2 | 40:60 |
| Preparation Example 2-3 | Organic alloy 3 | Compound 1-2 | Compound 2-3 | 40:60 |
| Preparation Example 2-4 | Organic alloy 4 | Compound 1-3 | Compound 2-4 | 40:60 |
| Preparation Example 2-5 | Organic alloy 5 | Compound 1-4 | Compound 2-1 | 40:60 |
| Preparation Example 2-A | Organic alloy A | Compound A-1 | Compound 2-1 | 40:60 |
| Preparation Example 2-B | Organic alloy B | Compound A-2 | Compound 2-1 | 40:60 |
| Preparation Example 2-C | Organic alloy C | Compound 1-1 | Compound B-1 | 40:60 |
| Preparation Example 2-D | Organic alloy D | Compound 1-4 | Compound B-2 | 40:60 |

### [Example: Manufacture of Organic Light Emitting Device]

### Example 1

A glass substrate on which a thin film of ITO (indium tin oxide) was coated in a thickness of 1400 Å was put into distilled water containing the detergent dissolved therein and washed by the ultrasonic wave. In this case, the used detergent was a product commercially available from Fischer Co. and the distilled water was one which had been twice filtered by using a filter commercially available from Millipore Co. The ITO was washed for 30 minutes, and ultrasonic washing was then repeated twice for 10 minutes by using distilled water. After the washing with distilled water was completed, the substrate was ultrasonically washed with isopropyl alcohol, acetone, and methanol solvent, and dried, after which it was transported to a plasma cleaner. Then, the substrate was cleaned with oxygen plasma for 5 minutes, and then transferred to a vacuum evaporator.

On the ITO transparent electrode thus prepared, 95 wt% of the following compound HT-A and 5wt% of the following compound PD were thermally vacuum-deposited to a thickness of 100 Å to form a hole injection layer, and then only the compound HT-A was deposited to a thickness of 1150 Å to form a hole transport layer. The following compound HT-B was thermally vacuum-deposited to a thickness of 450 Å thereon to form an electron blocking layer.

Then, the organic alloy 1 prepared in Preparation Example 2-1 and the following compound GD as a dopant material were vacuum-deposited in a weight ratio of 92:8 to a thickness of 350 Å on the electron blocking layer to form a light emitting layer.

Then, the following compound ET-A was vacuum-deposited to a thickness of 50 Å as a hole blocking layer. Then, the following compound ET-B and the following compound Liq were thermally vacuum-deposited in a ratio of 1:1 to a thickness of 300 Å as an electron transport layer, and then Yb was vacuum-deposited to a thickness of 10 Å as an electron injection layer.

Magnesium and silver were deposited in a weight ratio of 1: 4 to a thickness of 150 Å on the electron injection layer to form a cathode, thereby completing the manufacture of an organic light emitting device.

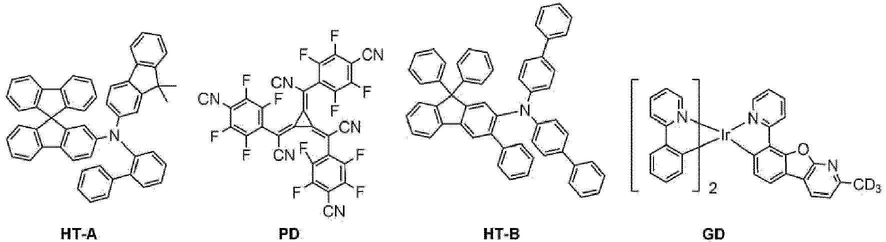

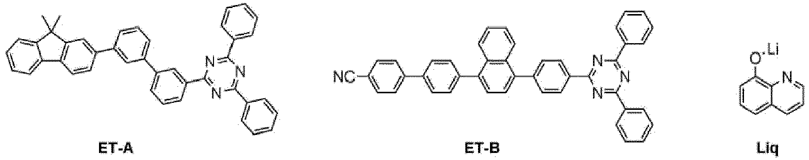

In the above-mentioned process, the vapor deposition rate of the organic material was maintained at 0.4 ~ 0.7 Å/sec, the deposition rate of magnesium and silver was maintained at 2 Å/sec, and the degree of vacuum during the deposition was maintained at 2*10⁻⁷ ~ 5*10⁻⁶ torr, thereby manufacturing an organic light emitting device.

### Examples 2 to 5, and Comparative Examples 1-1 to 3-4

The organic light emitting devices of Examples 2 to 5 and Comparative Examples 1-1 to 3-4 were manufactured in the same manner as in Example 1, except that the host material was changed as shown in Table 2 below. Wherein, in Comparative Examples 2-1 to 3-4, a simple mixture of two types of compounds was used as a host.

### [Experimental Example: Evaluation of Device Characteristics]

The organic light emitting devices manufactured in Examples 1 to 5 and Comparative Examples 1-1 to 3-4 were heat-treated in an oven at 120°C for 30 minutes, then taken out, and the voltage, efficiency, and lifetime (T95) were measured by applying a current, and the results are shown in Table 2 below. At this time, the voltage and efficiency were measured by applying a current density of 10 mA/cm², and T95 means the time (hr) required for the luminance to be reduced to 95% of the initial luminance at a current density of 20 mA/cm².

**[Table 2]**

| | Host material | @ 10 mA/cm² | | @ 20 mA/cm² |
|---|---|---|---|---|
| | | Voltage (V) | Efficiency (cd/A) | Lifetime (T95, hr) |
| Example 1 | Organic alloy 1 | 4.08 | 73.9 | 159 |
| Example 2 | Organic alloy 2 | 4.07 | 72.6 | 159 |
| Example 3 | Organic alloy 3 | 4.01 | 74.2 | 162 |
| Example 4 | Organic alloy 4 | 4.03 | 73.6 | 153 |
| Example 5 | Organic alloy 5 | 4.08 | 72.9 | 170 |
| Comparative Example 1-1 | Organic alloy A | 5.16 | 56.3 | 60 |
| Comparative Example 1-2 | Organic alloy B | 4.56 | 61.0 | 72 |
| Comparative Example 1-3 | Organic alloy C | 4.98 | 54.1 | 83 |
| Comparative Example 1-4 | Organic alloy D | 4.83 | 65.4 | 93 |
| Comparative Example 2-1 | Simple mixture of Compound 1-1:Compound 2-1 in a weight ratio of 40:60 | 4.12 | 69.7 | 102 |
| Comparative Example 2-2 | Simple mixture of Compound 1-2:Compound 2-2 in a weight ratio of 40:60 | 4.13 | 68.2 | 94 |
| Comparative Example 2-3 | Simple mixture of Compound 1-3:Compound 2-3 in a weight ratio of 40:60 | 4.09 | 68.3 | 113 |
| Comparative Example 2-4 | Simple mixture of Compound 1-3:Compound 2-4 in a weight ratio of 40:60 | 4.08 | 70.4 | 108 |
| Comparative Example 2-5 | Simple mixture of Compound 1-4:Compound 2-1 in a weight ratio of 40:60 | 4.12 | 68.2 | 109 |
| Comparative Example 3-1 | Simple mixture of Compound A-1 :Compound 2-1 in a weight ratio of 40:60 | 5.18 | 55.6 | 57 |
| Comparative Example 3-2 | Simple mixture of Compound A-2:Compound 2-1 in a weight ratio of 40:60 | 4.58 | 60.7 | 69 |
| Comparative Example 3-3 | Simple mixture of Compound 1-1 :Compound B-1 in a weight ratio of 40:60 | 5.01 | 53.6 | 80 |
| Comparative Example 3-4 | Simple mixture of Compound 1-4:Compound B-2 in a weight ratio of 40:60 | 4.89 | 64.2 | 88 |

It was confirmed that when an organic alloy was prepared using the compound represented by Chemical Formula 1 and the compound represented by Chemical Formula 2 and utilized as a host for the light emitting layer of an organic electroluminescent device, it exhibits low-voltage and high-efficiency characteristics as compared to materials having a structure different from Chemical Formula 1 or Chemical Formula 2, and that when an organic alloy was prepared with these compounds, it exhibits the characteristics of low voltage, high efficiency and long lifetime as compared to the case using a simple mixture.

### <Description of Symbols>

| | | | |
|---|---|---|---|
| 1: | substrate | 2: | anode |
| 3: | light emitting layer | 4: | cathode |
| 5: | hole injection layer | 6: | hole transport layer |
| 7: | electron blocking layer | 8: | hole blocking layer |
| 9: | electron transport layer | 10: | electron injection layer |

## Claims

1. An organic light emitting device comprising:
an anode;
a cathode; and
a light emitting layer interposed between the anode and the cathode,
wherein the light emitting layer includes an organic alloy of a compound represented by the following Chemical Formula 1 and a compound represented by the following Chemical Formula 2: 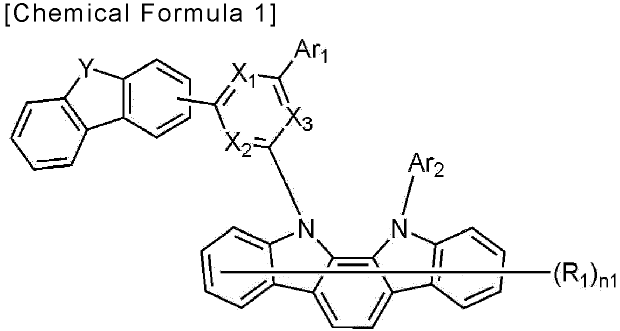 in Chemical Formula 1,
X₁, X₂ and X₃ are each independently CH or N, provided that at least one of X₁, X₂ and X₃ is N,
Ar₁ and Ar₂ are each independently a substituted or unsubstituted C₆₋₆₀ aryl; or a substituted or unsubstituted C₂₋₆₀ heteroaryl containing any one or more selected from the group consisting of N, O and S,
Y is O, S, or CRR',
wherein R and R' are each independently a substituted or unsubstituted C₁₋₆₀ alkyl,
R₁ is hydrogen; deuterium; a substituted or unsubstituted C₆₋₆₀ aryl; or a substituted or unsubstituted C₂₋₆₀ heteroaryl containing at least one selected from the group consisting of N, O and S, and
n1 is an integer of 1 to 10, 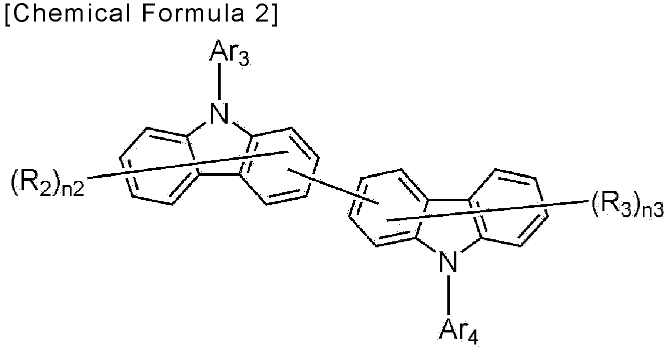 in Chemical Formula 2,
Ar₃ and Ar₄ are each independently a substituted or unsubstituted C₆₋₁₂ aryl; or a substituted or unsubstituted C₂₋₁₂ heteroaryl containing any one or more selected from the group consisting of N, O and S,
R₂ and R₃ are each independently hydrogen; deuterium; a substituted or unsubstituted C₆₋₆₀ aryl; or a substituted or unsubstituted C₂₋₆₀ heteroaryl containing any one or more selected from the group consisting of N, O and S,
provided that at least one of Ar₃ and Ar₄ is substituted with at least one deuterium, or at least one of R₂ and R₃ is deuterium, and
n2 and n3 are each independently an integer of 1 to 7.

2. The organic light emitting device according to claim 1, wherein:
Ar₁ is phenyl, biphenylyl, terphenylyl, or phenyl substituted with 5 deuteriums.

3. The organic light emitting device according to claim 1, wherein:
Ar₂ is phenyl or biphenylyl.

4. The organic light emitting device according to claim 1, wherein:
R and R' are each independently methyl.

5. The organic light emitting device according to claim 1, wherein:
R₁ is hydrogen or deuterium.

6. The organic light emitting device according to claim 1, wherein:
the compound represented by Chemical Formula 1 is any one selected from the group consisting of:

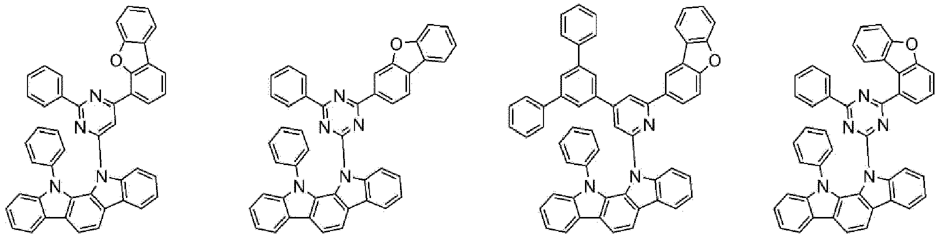

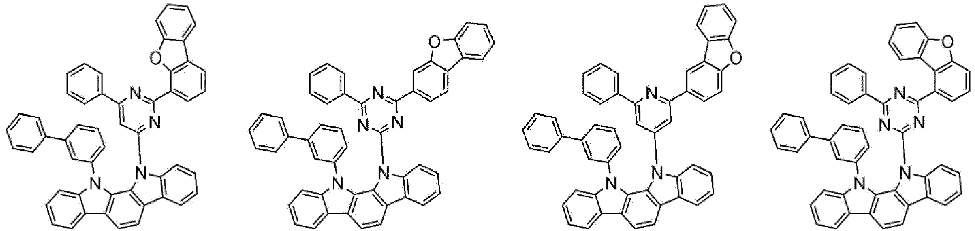

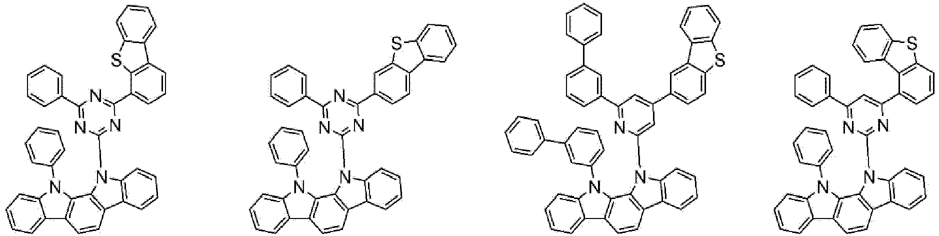

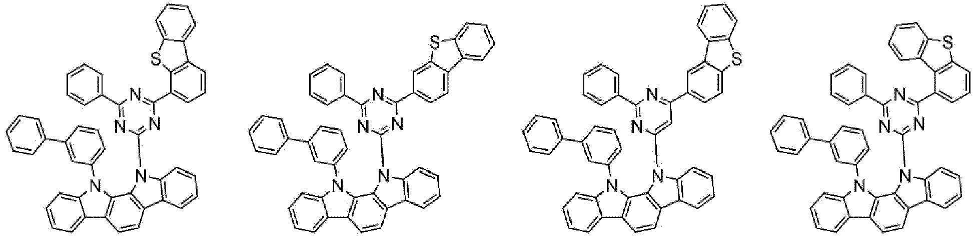

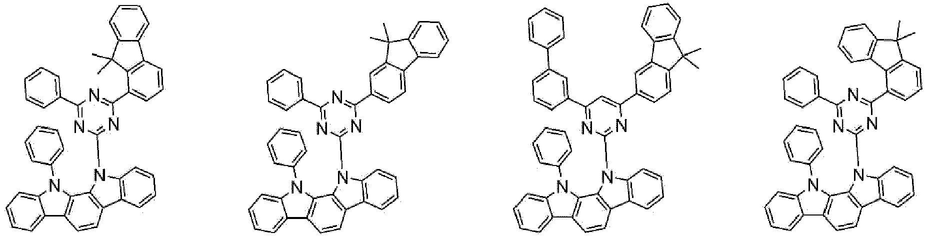

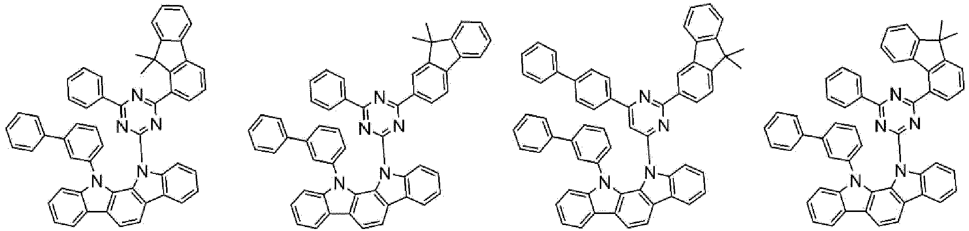

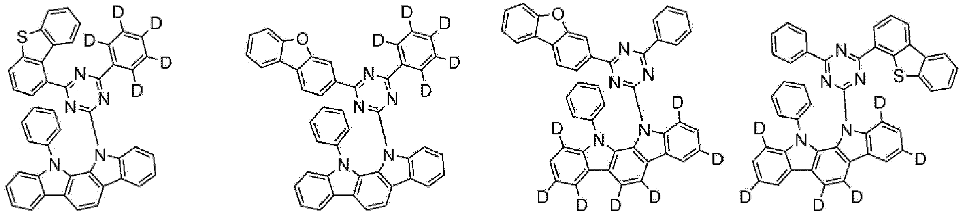

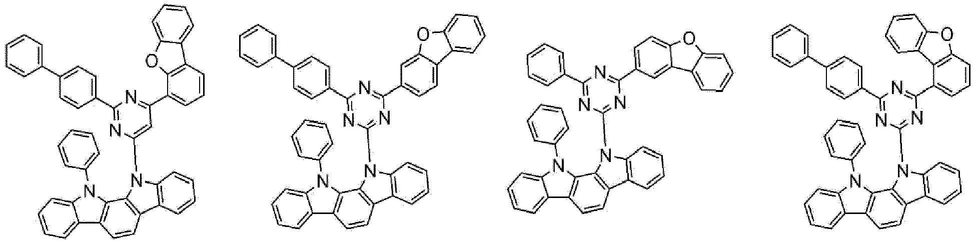

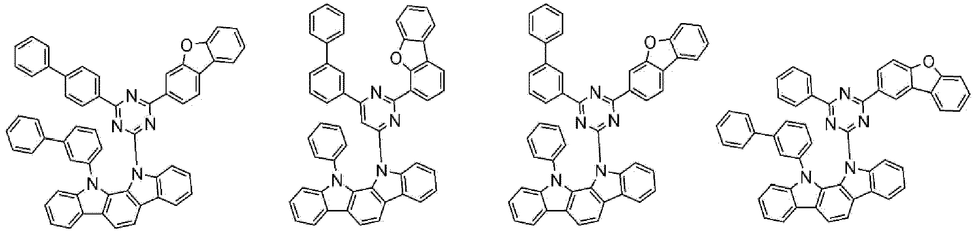

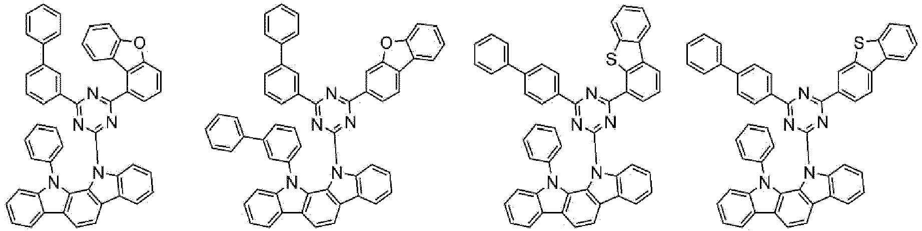

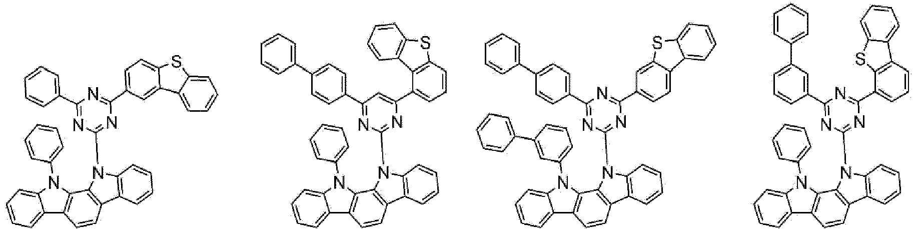

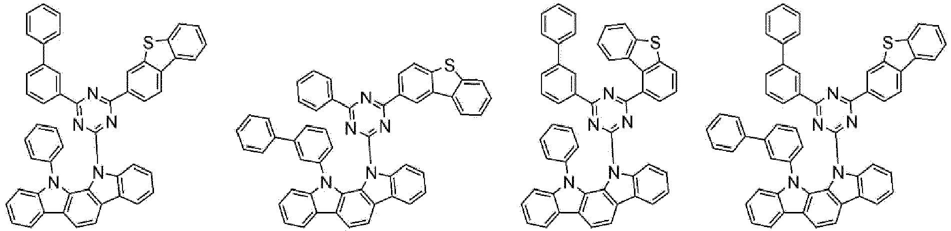

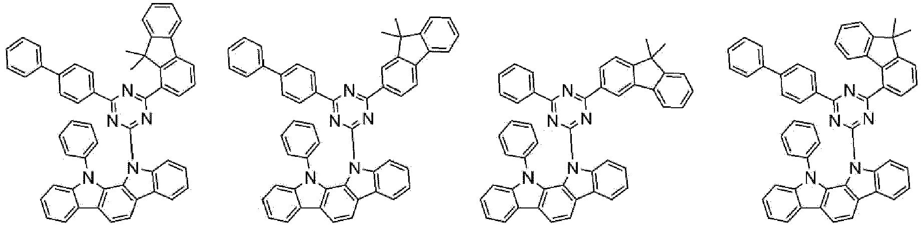

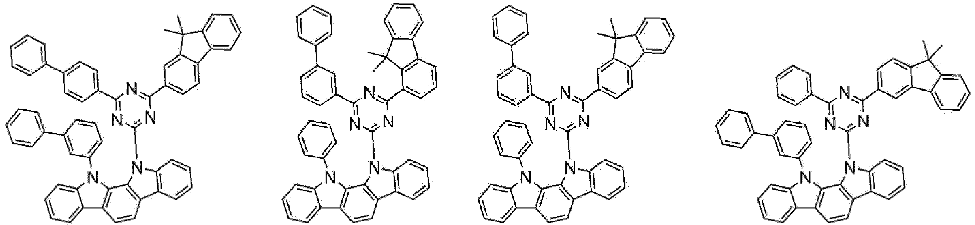

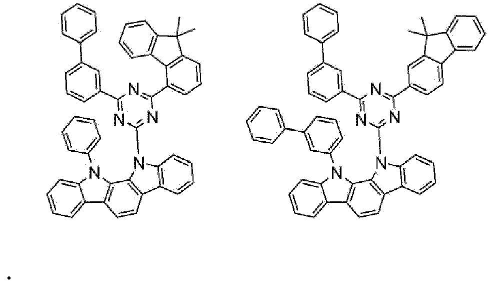

7. The organic light emitting device according to claim 1, wherein:
the compound represented by Chemical Formula 2 is represented by the following Chemical Formula 2-1: 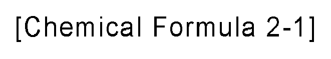 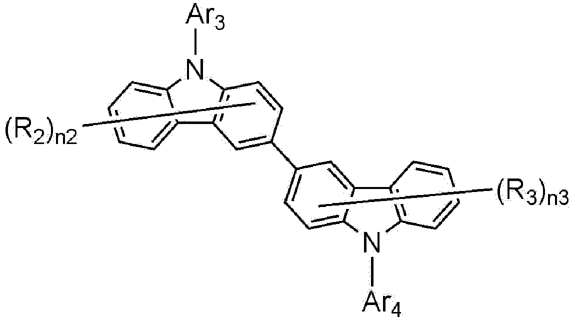 wherein, in Chemical Formula 2-1,
Ar₃, Ar₄, R₂, R₃, n2 and n3 are as defined in claim 1.

8. The organic light emitting device according to claim 1, wherein:
Ar₃ and Ar₄ are each independently phenyl, biphenylyl, naphthyl, dimethylfluorenyl, dibenzofuranyl, or dibenzothiophenyl,
the phenyl, biphenylyl, naphthyl, dimethylfluorenyl, dibenzofuranyl, or dibenzothiophenyl is unsubstituted or substituted with deuterium.

9. The organic light emitting device according to claim 1, wherein: R₂ and R₃ are each independently hydrogen, deuterium, or phenyl, the phenyl is unsubstituted or substituted with 1 to 5 deuteriums.

10. The organic light emitting device according to claim 1, wherein:
the compound represented by Chemical Formula 2 is any one selected from the group consisting of: 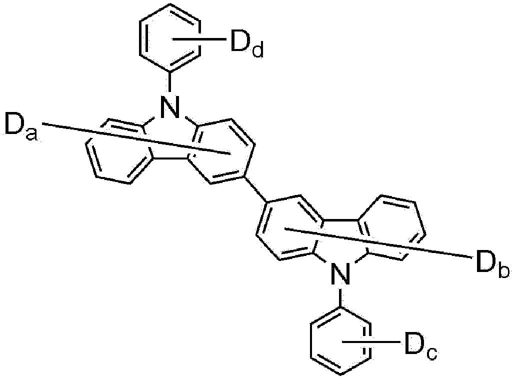 in the above compound,
a + b + c + d is 1 to 24, 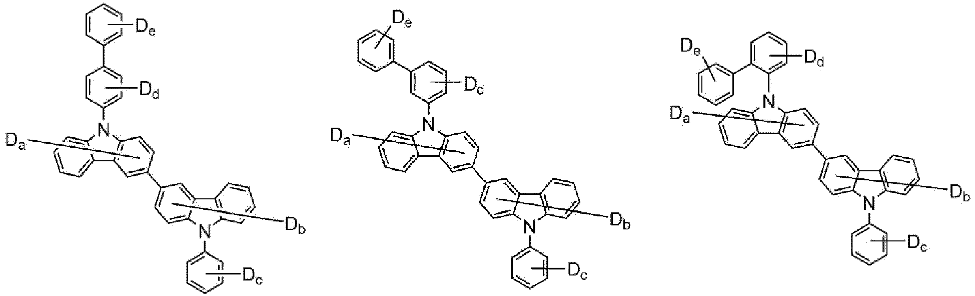 in the above group,
a + b + c + d + e is 1 to 28, 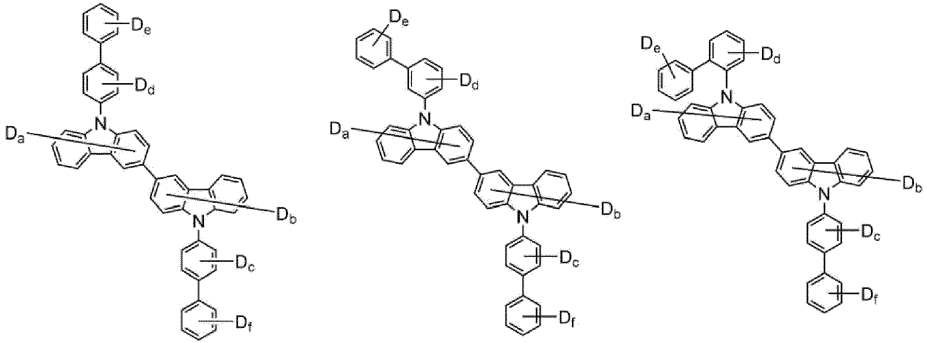 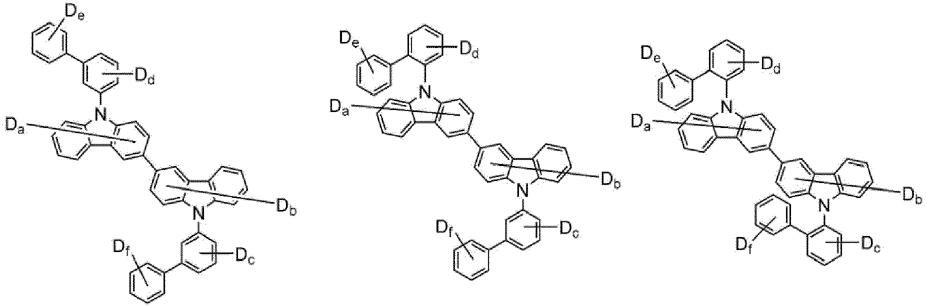 in the above group,
a + b + c + d + e + fis1 to 32, 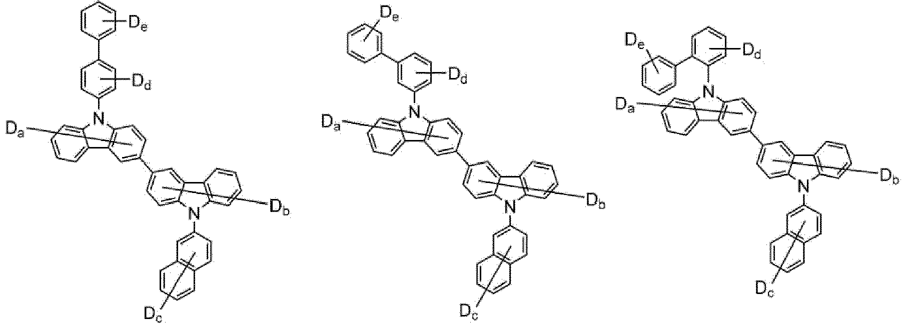 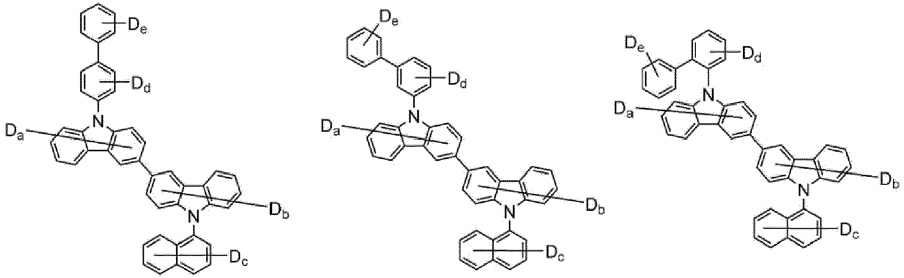 in the above group,
a + b + c + d + e is 1 to 30, 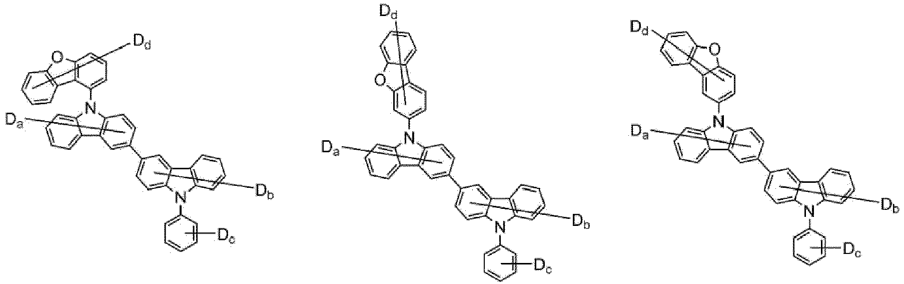 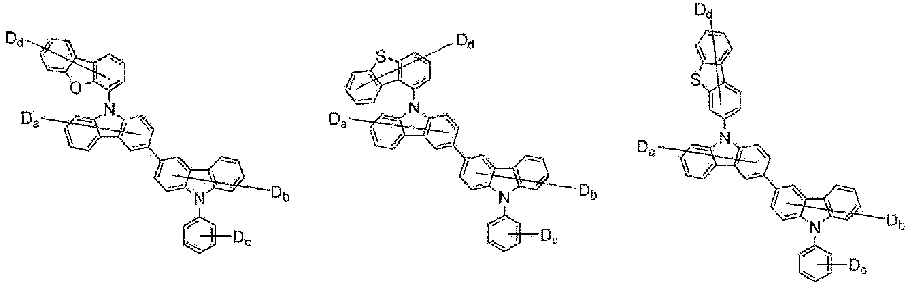 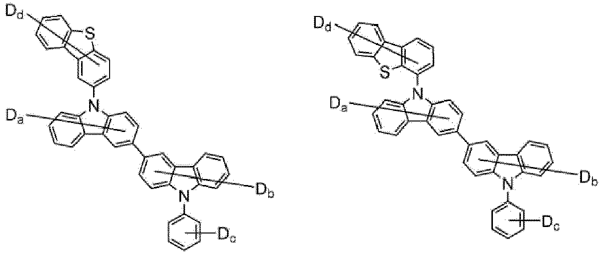 in the above group,
a + b + c + d is 1 to 26, 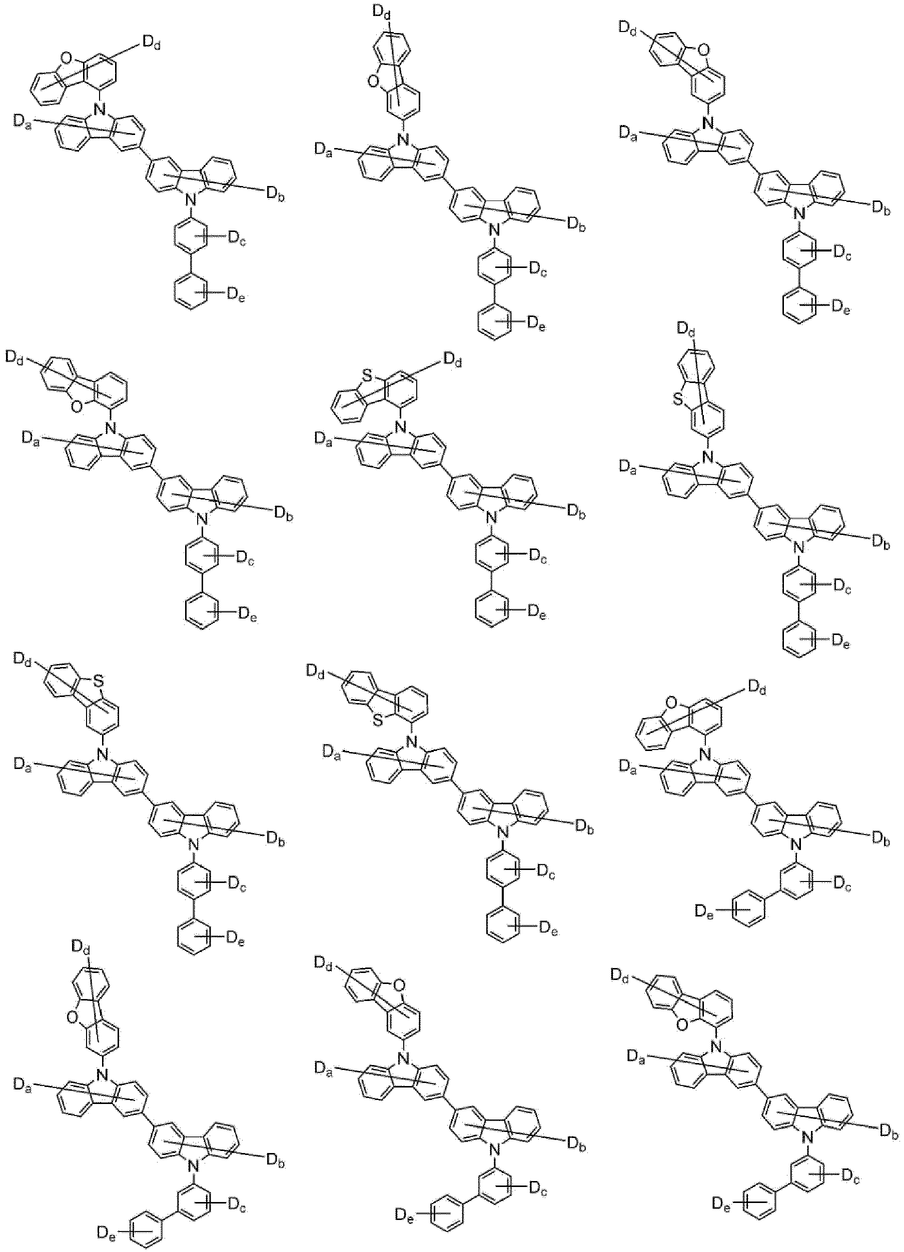 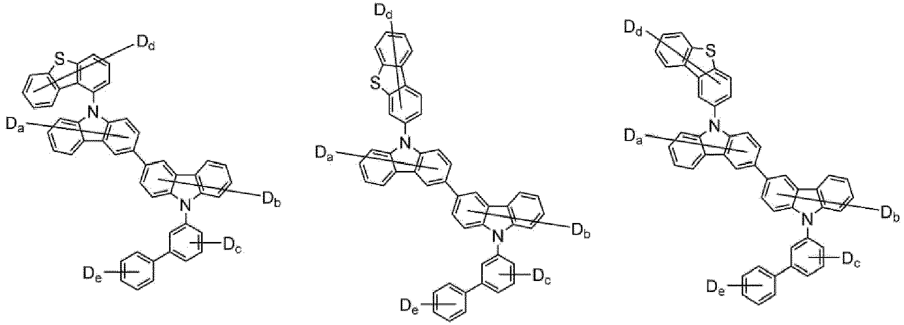 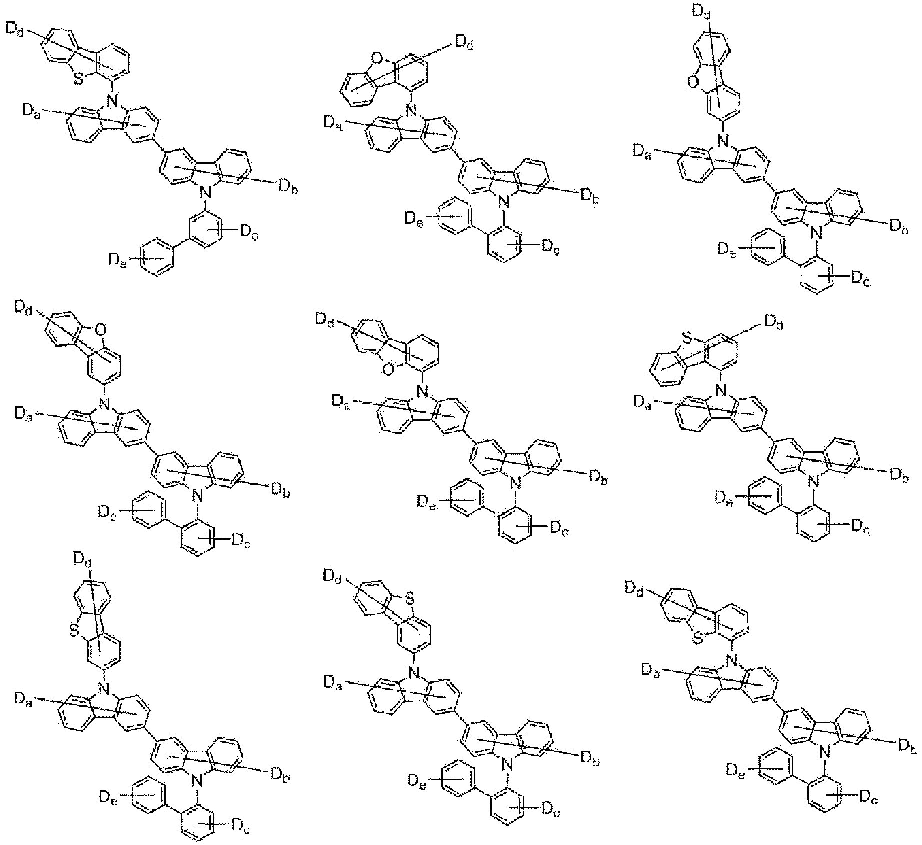 in the above group,
a + b + c + d + e is 1 to 30, 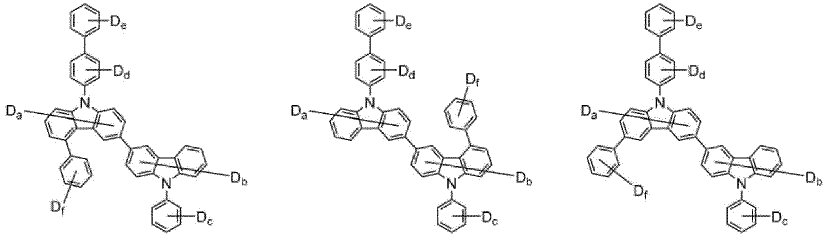 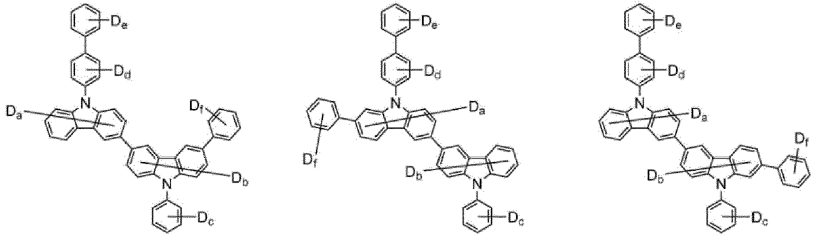 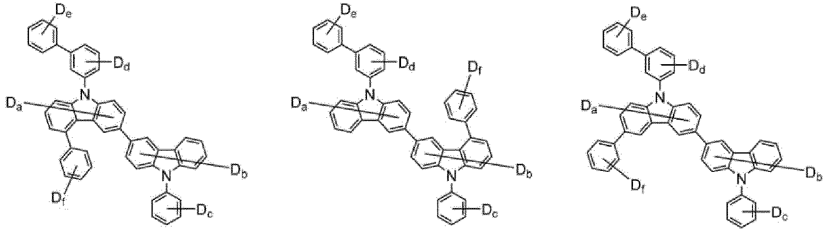 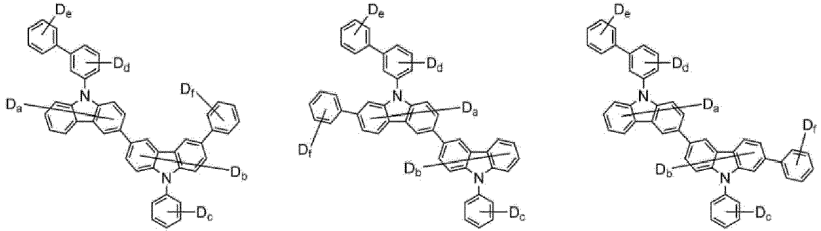 in the above group,
a + b + c + d + e + fis1 to 32, 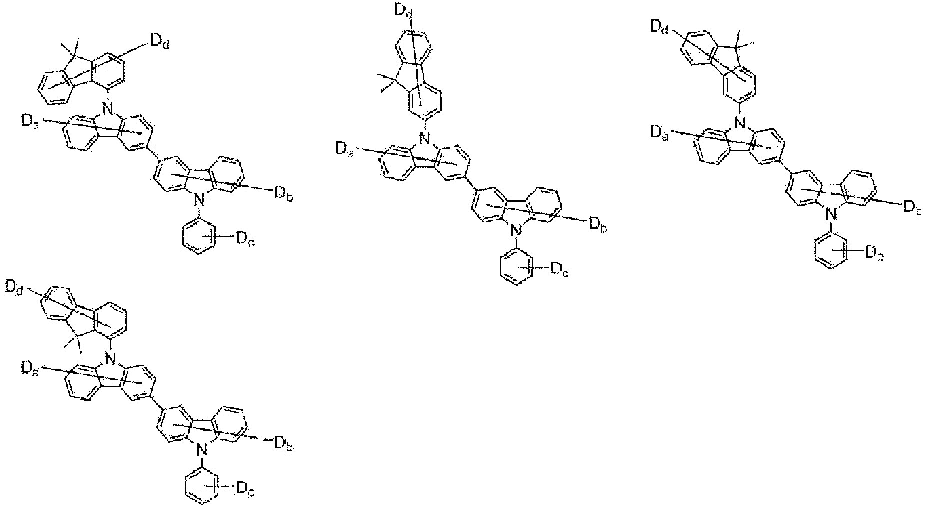 in the above group, a + b + c + d is 1 to 32, 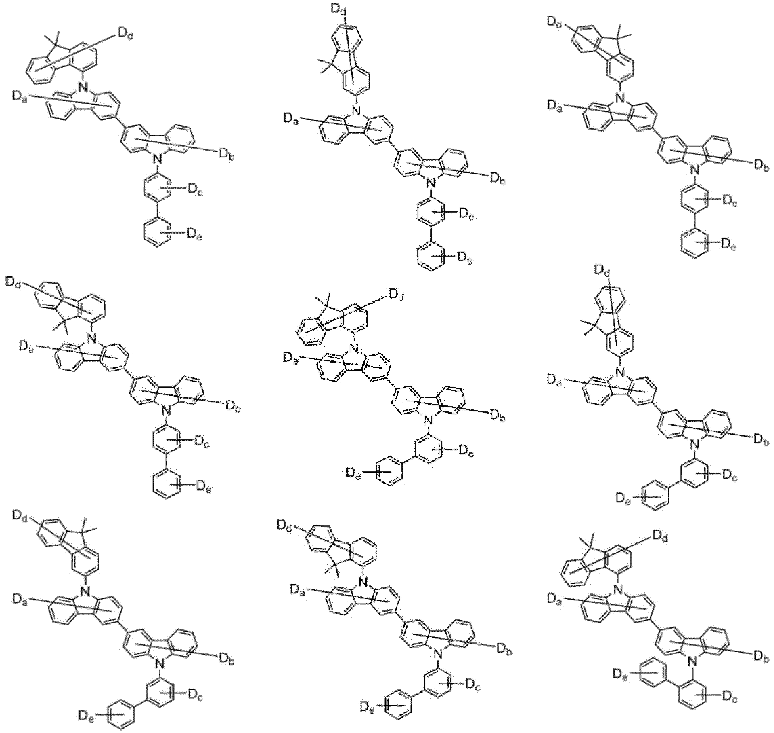 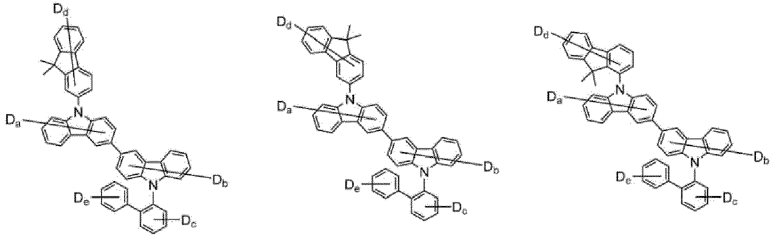 in the above group,
a + b + c + d + e is 1 to 36.
